(19) Europäisches Patentamt
European Patent Office
Office européen des brevets

(11) EP 1 336 847 B1

## (12) EUROPEAN PATENT SPECIFICATION

(45) Date of publication and mention of the grant of the patent:
**12.12.2007 Bulletin 2007/50**

(51) Int Cl.:
***G01N 33/50*** *(2006.01)* ***A61K 38/57*** *(2006.01)*
***C12N 15/11*** *(2006.01)* ***C12Q 1/68*** *(2006.01)*
***G01N 33/573*** *(2006.01)*

(21) Application number: **02003400.5**

(22) Date of filing: **14.02.2002**

# (54) Cathepsin Y inhibitors for the development of a medicament for the treatment of pain

Kathepsin Y Inhibitoren für die Entwicklung von Medikamenten zur Schmerzbehandlung

Inhibiteurs de la Cathepsine Y pour le développement de médicaments pour le traitement de la douleur

(84) Designated Contracting States:
**AT BE CH CY DE DK ES FI FR GB GR IE IT LI LU MC NL PT SE TR**

(43) Date of publication of application:
**20.08.2003 Bulletin 2003/34**

(73) Proprietor: **Biofrontera Pharmaceuticals AG**
**51377 Leverkusen (DE)**

(72) Inventors:
- **Lübbert, Hermann, Prof. Dr.**
  **51381 Leverkusen (DE)**
- **Schmitz, Beate, Dr.**
  **50825 Köln (DE)**

(74) Representative: **Polypatent**
**Postfach 40 02 43**
**51410 Bergisch Gladbach (DE)**

(56) References cited:
**WO-A-01/23570**
**WO-A-96/39194**
**WO-A-99/31256**

- **DEUSSING JAN ET AL: "Murine and human cathepsin Z: cDNA-cloning, characterization of the genes and chromosomal localization." BIOCHIMICA ET BIOPHYSICA ACTA, vol. 1491, no. 1-3, 25 April 2000 (2000-04-25), pages 93-106, XP002216940 ISSN: 0006-3002**
- **DATABASE BIOSIS [Online] BIOSCIENCES INFORMATION SERVICE, PHILADELPHIA, PA, US; 2000 PUNGERCAR JOZE ET AL: "Identification and molecular cloning of cathepsin P, a novel human putative cysteine protease of the papain family." Database accession no. PREV200000240928 XP002216941 & PFLUEGERS ARCHIV EUROPEAN JOURNAL OF PHYSIOLOGY, vol. 439, no. 3 Suppl., 2000, pages R116-R118, 1998 Life Sciences Conference: Signalling Concepts in Life Sciences.;Godz Martuljek, Slovenia; September 19-24, 1998 ISSN: 0031-6768**
- **NAGLER D K ET AL: "Human cathepsin X: A novel cysteine protease of the papain family with a very short proregion and unique insertions" FEBS LETTERS, ELSEVIER SCIENCE PUBLISHERS, AMSTERDAM, NL, vol. 434, no. 1-2, 28 August 1998 (1998-08-28), pages 135-139, XP004258274 ISSN: 0014-5793**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 336 847 B1

## Description

The present invention relates to the use of a polynucleotide sequence encoding Cathepsin Y or a amino acid sequence of Cathepsin Y protein for the characterisation or identification of therapeutical agents for pain, the use of such sequences for the development of a medicament, and agents useful as medicaments for the treatment of pain.

The effective treatment of pain requires an understanding of its physiology. It is well known, however, that stimuli which activate pain receptors in one tissue may not activate pain receptors in another. For example, pricking or cutting which causes pain in skin tissue does not cause pain in the stomach or intestine. The causes of pain in skeletal muscle, joints, and arteries can also differ. Principles of Neurology, 6'"ed., Adams, R. D., et al., eds. (McGraw-Hill: 1997), pp. 133-134. Consequently, methods useful for relieving one type of pain are often less effective, or even ineffective, when applied to the alleviation of others. In general, neuropathic pain is persistent and is characterised by burning, gnawing, aching, shooting, or lancinating sensations. It is frequently associated with hyperesthesia, hyperalgesia, allodynia, and hyperpathia, and in some cases by sensory deficit or autonomic dysfunction. Unfortunately, and unlike other types of pain, neuropathic-pain tends to respond poorly to analgesic medication. Principles of Neurology, 6''ed, Adams, R. D., et al., eds. (McGraw-Hill: 1997), p. 140.

Depending on the nerves involved, a particular instance of neuropathic pain can be classified as a central or peripheral neuropathy. Central neuropathies arise from spinal cord, brainstem, thalamic, and cerebral damage or disease, while peripheral neuropathies arise from damage or disease of peripheral nerves. Specific peripheral neuropathies include, but are not limited to: thoracic outlet obstruction syndromes; compression and entrapment neuropathies such as ulnar nerve palsy, carpal tunnel syndrome, peroneal nerve palsy, radial nerve palsy; and Guillain-Barré syndrome. The Merck Manual, 16th ed., 1518-1522 (1992).

Neuropathic, or neurogenic, pain arises from the direct stimulation of nervous tissue. Neuropathic pain encompasses a wide variety of disorders involving single and multiple nerves. These include, but are not limited to, trigeminal neuralgia and disorders due to herpes zoster, diabetes, and trauma (including causalgia); spinal arachnoiditis and spinal cord injuries; and the thalamic pain syndrome of Dejerine-Roussy. Principles of Neurology, ah ed., Adams, R. D., et al., eds. (McGraw-Hill: 1997), p. 140.

Neuropathic pain is caused by a variety of factors including, but not limited to: trauma caused by injury or surgical operation; tumors; bony hyperostosis; casts; crutches; prolonged cramped postures; hemorrhage into a nerve; exposure to cold or radiation; collagen-vascular disorders; infectious diseases such as Lyme disease and HIV; toxins such as emetine, hexobarbital, barbital, chlorobutanol, sulfonamides, phenytoin, nitrofurantoin, the vinca alkaloids, heavy metals, carbon monoxide, triorthocresylphosphate, orthodinitrophenol, and other solvents and industrial poisons; autoimmune reactions; nutritional deficiency, and vitamin B deficiency in particular; and metabolic disorders such as hypothyroidism, porphyria, sarcoidosis, amyloidosis, uremia and diabetes. The Merck Manual, 16th ed., 1518 (1992).

Because so many causes of neuropathic pain exist, and because it tends to respond poorly to analgesic medication, the discovery of drugs that safely and effectively aid in its relief has been difficult.

Whereas acute pain is generally symptomatic of tissue damage or inflammation, and lasts only as long as the underlying cause remains, chronic pain may persist indefinitely in the absence, or after recovery of tissue pathology. It may occur as the aftermath of injury (e.g. to peripheral nerves or to the spinal cord), or in association with other disease states, such as herpes zoster or diabetes, but often it arises with no obvious cause. Chronic pain is often severe, leading to major disability and a high suicide rate, and it is common, with a prevalence of approximately 1% at the severe level. In contrast to acute pain, chronic pain is often unresponsive to conventional analgesic drugs (opiates and NSAIDs), and presents a difficult therapeutic problem, since its cause can usually not be resolved. Various other classes of drug, (e.g. tricyclic antidepressants, some anticonvulsant drugs such as gabapentin) may be effective, but the therapeutic response is very variable.

A common feature of many clinical syndromes where chronic pain develops is the existence of previous nerve damage, affecting peripheral nerves, the spinal cord or (as in stroke) the brain, and the concept of neuropathic pain (i.e. pain arising as a consequence of neuronal damage) has become accepted as the underlying cause of many different chronic pain conditions seen in the clinic. Several animal models of neuropathic pain have been developed, which mimic many aspects of the clinical condition. These include lesions of the sciatic nerve (constriction or partial section), section of spinal nerves, ischaemic lesions of the spinal cord, diabetic neuropathy, etc. and such models have been subjected to detailed study of the anatomical, biochemical and physiological changes that accompany the development of the pain state.

In the last several years a number of experimental models for neuropathic pain have been developed (Bennett and Xie (1988), Pain 33:87-107; Seltzer et al. (1990), Pain 43:205-218; Kim and Chung (1992), Pain 50:355-363; DeLeo et al. (1994) Pain 56:9-16; Na et al. (1994), Neurosci. Lett. 177:50-52). The availability of these different models provides an opportunity to investigate mechanisms of neuropathic pain. Finding common features in different models should provide better insight into the mechanisms critical for neuropathic pain, and comparison of the models should help to understand pain development and progression. for neuropathic pain, and comparison of the models should help to

understand pain development and progression.

Kim et al. compared three of the models on basis of neuropathic pain behaviours and the effects of surgical sympathectomy (Kim et al. (1997), Exp. Brain Res. 113:200-206). They found that the models of Bennett, Seltzer and Kim & Chung (reference see above) result in a very similar general pattern and time course of evoked pain behaviour, whereby the Bennett model showed biggest behavioural signs for ongoing pain. The same results have been shown for the effects of sympathectomy on the behavioural signs of evoked and ongoing neuropathic pain, respectively. Thus these three models can be used to discover basic common features involved in neuropathic pain.

Further animal models for pain are considered in an article of Walker et al. (1999), Molecular Medicine Today 5:319-321, comparing models for different types of pain, which are acute pain, chronic / inflammatory pain and chronic / neuropathic pain, on the basis of behavioural signs.

WO 99/31256 discloses cathepsin DC polypeptides, which are homologues of cathepsin Y according to the present application, the nucleic acid encoding such polypeptides, the encoded proteinases, antibodies generated against these polypeptides, fragmented peptides derived from these polypeptides, antisense oligonucleotides and the uses of the before mentioned for human cancer prognosis.

Jan Deussing et al. describe in Biochimica et Biophysica Acta (2000), 93-106, murine and human cathepsins Z, their cDNA cloning, characterization of the genes and chromosomal localization. The cathepsins Z show a high homology with homologues from other species, particularly prognosted three-dimensional structure is discussed.

Pungercar, J. and Ivanovski, G. disclose in Pfluegers Archiv European Journal of Physiology, Vol. 439, No.3 (2000), page R116-R118, an abstract of identification and molecular cloning of cathepsin P, a novel human putative cysteine protease of papain family, which was part of Life Science Conference 1998.

Nägler, D. and Ménard, R. discuss in FEBS Letters 434 (1998) 135-139, a novel cysteine protease of the papain family (human cathepsin X) with a short proregion and unique insertions, whereby the sequence is obtained by PCR amplification from a human ovary cDNA library. Sequence of DNA and predicted protein is discussed, as well as homology of the cathepsin X with prior known cathepsins.

In WO 01/23570 new voltage-gated sodium channels are disclosed, whereby the protein is a splice variant of the $\beta$ subunit of sodium channels. These nucleic acids and proteins are used in screening methods for the identification of compounds that modulate the expression of the nucleic acid an proteins for the treatment of neuropathic pain.

Object of the present invention is to provide a target for examination and development of a medicament for the treatment of pain.

This object is met by a method for the development of a medicament for the treatment of pain comprising the use of a polynucleotide sequence selected from SEQ ID NO:1, 2 or 4 encoding Cathepsin Y or homologues or fragments thereof comprising at least 10 identical base pairs or the according protein / polypeptide, comprising determination of the efficiency of a compound or agent in a model for pain, whereby the efficiency of the compound / agent is determined by considering expression rate of the gene or by considering activity of the protein.

According to the invention it has been found that Cathepsin Y is differentially expressed under pain, particularly under neuropathic pain. In the present invention it is shown that in three different rat models Cathepsin Y expression is upregulated under neuropathic pain. Examinations have been carried out in the models of Bennett, Seltzer and Kim & Chung

The model of Bennett is based on the chronic constriction injury by the loose ligation of the sciatic nerve. Therefore this model is designated as "CCI model".

The model of Seltzer et al. is based on the tight ligation of the partial sciatic nerve and is therefore designated as "PSL model".

The model of Kim & Chung is based on the tight ligation of spinal nerves and is therefore designated as "SNL model".

These designations correspond to the designations used in the cited literature.

The three models are explained more in detail in the literature and in the examples below.

The term "polynucleotide sequence" or "nucleic acid sequence" designates in the present application any DNA or RNA sequence, independent of the length. Thus this term can describe short sequences like PCR primers or probes for hybridisation, as well as whole genes or cDNA of these genes.

The term "polypeptide" or "amino acid sequence" designates a chain of amino acids, independent from their length, but in any case more than one amino acid.

As "homologues" of polynucleotide sequences such polynucleotide sequences are designated which encode the same type of protein as the polynucleotide sequence described herein, particularly a homologous polynucleotide sequence that encodes a polypeptide with the same type of activity. Accordingly as "homologues" of a polypeptide polypeptides are designated which have an amino acid sequence, wherein at least 70 %, preferably 80 %, more preferably 90 % of the amino acids are identical to the protein of the present invention and wherein the replaced amino acids preferably are replaced by homologous amino acids. As "homologous" amino acids are designated which have similar features concerning hydrophobicity, charge, steric features etc. Most preferred are amino acid sequences, containing

the species- or family-dependent differences of the amino acid sequence. Particularly as "homologues" sequences are designated those which correspond to one of the cited sequences in another species or individuum. For example if in the present invention a rat model is used and the cited polynucleotide sequence encodes the rat protein, the according polynucleotide sequence and protein of a mouse in a mouse model is designated as "homologue". Further, splice variants and members of gene families are designated as homologues.

"Fragments" of a polynucleotide sequence are all polynucleotide sequences which have at least 10 identical base pairs compared to the polynucleotide sequence shown in the present application or by the gene represented by these polynucleotide sequence. The term "fragment" encloses therefore such fragments as primers for PCR, probes for hybridisation, DNA fragments included in DNA vectors like plasmids, cosmids BACs or viral constructs, as well as shortened splice variants of the genes identified herein. As a fragment of a protein (polypeptide) amino acid sequences are designated which have at least three amino acids, preferably at least 10 amino acids. Therefore fragments serving as antigens or epitopes are enclosed in this designation.

In the present application the term "sequence" is used when either a polynucleotide sequence (= nucleic acid sequence) or a polypeptide (= amino acid sequence) or a protein is meant. That means when it is irrelevant which type of sequence is used the type is not designated particularly, but with the more common term "sequence".

The basis of the methods and assays described in the present application is the examination of Cathepsin Y which is differentially expressed under pain or during development of pain. For the examination necessary for the development of any medicament each sequence can be used which allows the determination of the expression rate of the Cathepsin Y gene or the activity of the polypeptide / protein. The considered sequence can be the polynucleotide sequences SEQ ID NO. 1, SEQ ID NO. 2 or SEQ ID NO. 4, respectively or homologues or fragments thereof, as well as the polypeptides encoded thereby (SEQ ID NO. 3 or SEQ ID NO. 5, respectively) or homologues or fragments thereof.

According to the invention it has been find out, that the Catepsin Y gene represented by the polynucleotide sequences SEQ ID No: 2 is differentially expressed correspondingly in three different models for pain, which are the above described models "CCI", "PSL", SNL".

Therefore the present invention provides a target for the development of medicaments for the treatment of pain, particularly for the treatment of neuropathic pain. Cathepsin Y has not yet been regarded in relation to pain.

JP2000157263 describes a human Cathepsin Y useful in the elucidation and therapy for Alzheimer's disease, neurodegenerative diseases, cancers and the like. Comparing the nucleic acid sequence shown in this publication with the sequences of common data bases, however, shows that the provided sequence corresponds to Cathepsin F, not to Cathepsin Y.

WO 96/39194 teaches that the Cathepsin Y protein is involved in the secretion of β-amyloid peptide (β-AP) from cells, resulting in the development of Alzheimer's disease. The nucleic acid sequence as well as the protein sequence of Cathepsin Y is provided. For the treatment of Alzheimer's disease the inhibition of this protein is proposed, therefore inhibitors of Cathepsin Y are examined.

According to the present invention the Cathepsin Y sequences (polynucleotide sequence or polypeptide) can be used in any test system which allows the determination of either the expression rate of the gene or of the activity of the polynucleotide sequence or of the polypeptide / protein.

For determination and comparison of the expression levels of at least one of the genes identified in the present invention any of the commonly known methods can be used, either on RNA/cDNA level or on protein level. For example PCR, hybridisation, micro array based methods, western blot or 2-D protein gel analysis are suitable methods. One preferred method is the digital expression pattern display method (DEPD method), explained in detail in WO99/42610. The method used for determination of expression levels is not restrictive, as long as expressed amounts can be quantified.

Activity of the Cathepsin Y protein can be determined by, contacting the protein with polypeptides under acid pH conditions and determining the concentration of free amino acids after cleavage. Activity of the Cathepsin Y protein is a carboxypeptidase activity. Methods for determination of Cathepsin Y activity are explained in detail in international patent application WO 96/39194.

Expression and activity of Cathepsin Y can further be determined in assay systems or models. Such assay systems may be in vivo, ex vivo or in vitro assays, for example a cellular assay system comprising cells expressing Cathepsin Y,or an assay comprising isolated Cathepsin Y.

In any assay or model at least one of the sequences is contacted with the compound(s) to be tested and samples are obtained, wherein expression levels or activity of the sequences are determined and compared to non-treatment conditions.

For examination of the expression rate of the gene, animal models can be used. As such a model any animal can be used wherein the necessary preparations can be carried out, however, mammalian models are preferred. Even more preferred are rodents and lagomorphae, particularly preferred are rats, mice and rabbits. The most preferred animal model of the present invention is a rat model.

Dependent from the model used, the samples can be derived from whole blood, cerebrospinal fluid (CSF) or whole tissue, from cell populations isolated from tissue, cerebrospinal fluid (CSF) or 6lood or from single cell populations

(i.e. cell lines).

In one embodiment of the invention cellular assays can be used. Preferred cells for cellular assays are eukaryotic cells, more preferably mammalian cells. Most preferred are neuronal-like cells, like SHSY5Y (neuroblastoma cell line) or COS cells (African green monkey, kidney cells); CHO cells (Chinese hamster ovary), HEK-293 cells (human embryonic kidney).

The Cathepsin Y sequences of the present invention can be used for diagnosing a pain status, particularly a neuropathic pain status of a human outside of the living body by determining the expression levels or activity of Cathepsin Y sequences in comparison to the non-disease status. During treatment period of a patient the expression or activity of the sequences can also be used for assessing the efficacy of pain treatment outside of the body. In these cases blood, CSF or tissue is removed from the patient and expression or activity is determined in the samples.

The disease which is considered in the present invention is pain. The preferred types of pain are persistent / central pain, inflammatory pain (acute or chronic) or neuropathic pain, particularly chronic neuropathic pain. The most preferred type is neuropathic pain.

Examples of such diseases are diabetic neuropathy, post-herpetic neuralgia, trigeminal neuralgia, cancer associated pain, spinal cord injury, multiple sclerosis, phantom pain, post-stroke pain, HIV associated pain, low back pain associated neuropathic pain, complex regional pain syndromes, like reflex symparenthic dystrophy and causalgia, myofacial syndromes or idiopathic pain conditions.

Independent whether the efficacy of pain treatment or the efficiency of a compound for the treatment of pain shall be examined or a pain status is diagnosed on characterised, determination of the expression level or the activity of the sequences is carried out outside of a living body. As mentioned above, detection of the expression of the genes can be carried out by any method known in the art. The method of detection is not limiting the invention.

Expression levels can be detected either on basis of a polynucleotide sequence or by detecting the according polypeptide, encoded by said polynucleotide sequence.

Preferred methods for detection and determination of the gene expression levels are PCR of cDNA, generated by reverse transcription of expressed mRNA, hybridisation of polynucleotides (Northern-, Southern Blot systems, *In situ* hybridisation), DNA-micro-array based technologies, detection of the according peptides or protein via e.g. Western Blot systems, 2-dimensional gel analysis, protein micro-array based technologies or quantitative assays like e.g. ELISA tests.

The most preferred method for quantitative analysis of the expression levels is the differential expression pattern display method (DEPD), described in detail in W099/42610.

By using any of the Cathepsin Y sequences (polynucleotide sequence or polypeptide / protein) the efficiency of compounds for reducing the expression or the activity of the polynucleotide sequence or the protein can be tested. Therefore the sequences of the present invention can be used for identifying therapeutical agents and their efficiency for the treatment of pain.

For example a method can be used comprising:

a) providing a test cell population comprising cells capable of expressing the Cathpsin Y gene(s) or homologues or fragments thereof

b) contacting said test cell population with the test therapeutic agent,

c) detecting the expression of the Cathepsin Y gene(s) in said test cell population,

d) comparing the expression of the gene(s) in the test cell population to the expression of the gene(s) in a reference cell population whose disease stage is known; and

e) identifying a difference in expression levels of the considered sequences, if present, in the test cell population and the reference cell population, thereby identifying a therapeutic agent for treating pain.

For this method an animal model can be used or test cells can be obtained from a subject, an animal model or cell cultures of fresh cells or cell lines. Further *in vitro* assays may be used.

Agents or compounds which have any influence on the expression rate of the Cathepsin Y gene(s) might be used as a medicament for the treatment of pain. A method examining the expression rate of the Cathepsin Y gene therefore can be used for testing agents and compounds for their efficiency for treatment of pain. Which model is used is not relevant, as long as the model allows to determine differences in expression amounts.

In such a model cells are contacted with the interesting agent or compound and expression of Cathepsin Y gene is determined in comparison to the expression in cells which never have been contacted to the according agent / compound. Contacting the cells either can be effected by administering the agent / compound to an animal or by contacting isolated cells of tissue or blood or cells of cell lines in culture with the agent / compound.

By examination of the influence the considered agent / compound has to the expression of the Cathepsin Y gene the efficacy of the agent / compound for treating pain can be estimated. This allows the decision whether it is worthwhile to develop a medicament containing such an agent or compound for the treatment of pain.

Whether the expression is determined on the basis of mRNA generation (transcription level) or on basis of

protein generation (translation level) is not relevant, as long as the difference of the expression rate can be determined. Therefore the polynucleotide sequence, as well as the polypeptide or protein shown in the present application can be used for the development of a medicament.

The development of a medicament can be desirable for example if the considered compound /agent have any influence on the regulation of the expression rate or on the activity of any polynucleotide sequence or polypeptide of the present invention. Particularly if the agent or compound decreases the expression rate or the activity of Cathepsin Y it might be a candidate for the development of a medicament.

Said influence of a compound or agent can be examined by a method comprising contacting a sample comprising the nucleic acid sequence or homologues or fragment thereof or the according polypeptides of the present invention with a compound that binds to said sequence in an amount sufficient to determine whether said compound modulates the expression rate or the activity of the polynucleotide or polypeptide sequence.

By such a method a compound or agent can be determined modulating the expression rate or activity of the nucleic acid sequence or homologues or fragments thereof or the according polypeptides.

A method for determination of the efficiency of a compound / agent for the treatment of pain by determination of the activity of the Cathepsin Y protein comprises for example

a) contacting the Cathepsin Y protein with a compound /agent in presence of a known polypeptide which is a target of the Cathepsin Y,
b) determining the aminoterminal amino acid of-the pepti de or the amount of free amino acids in the sample after incubation,
c) comparing the aminoterminal amino acid of the peptide or the amount of free amino acids with the result in a sample which doesn't contain the compound / agent.

If a compound / agent is able to decrease or inhibit the activity of the Cathepsin Y protein it might be useful as a medicament for the treatment of pain.

Examples for compounds which can decrease Cathepsin Y activity are compounds of formula I:
wherein:

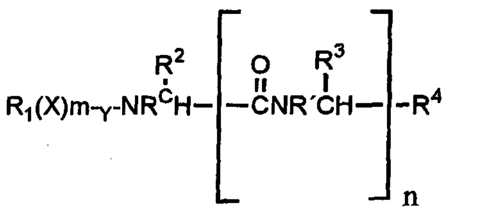

formula I

R is selected from the group consisting of hydrogen, alkyl of from 1 to 6 carbon atoms, and where R and R2 are joined to form a ring structure of from 4 to 10 carbon atoms,
R' is selected from the group consisting hydrogen, alkyl of from 1 to 6 carbon atoms and where R' and $R^3$ are joined to form a ring structure of from 4 to 10 carbon atoms,
$R^1$ is selected from the group consisting of alkyl of from 1 to 4 carbon atoms substituted with from 1 to 5 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur
wherein said substituted alkyl group is optionally further substituted with from 1 to 2 hydroxyl groups,
alkenyl of from 2 to 4 carbon atoms substituted with from 1 to 4 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur,
aryl of from 6 to 10 carbon atoms,
aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6

to 10 carbon atoms, hydroxy, cyano, halo and amino, fluorenyl,
heterocycles of from 3 to 14 carbon atoms having from 1 to 3 heteroatoms selected from the group consisting of nitrogen, oxygen and sulfur;
$R^2$ and $R^3$ are independently D- or L-amino acid side chains of at least 2 carbon atoms with the proviso that said amino acid side chains do not include the proline side chain;
$R^4$ is selected from the group consisting of
-C(O)CH =N=N, -$CH_2$OH, -C=NOH, and -C(O)$R^5$
where $R^5$ is hydrogen, alkyl of from 1 to 6 carbon atoms, haloalkyl of from 1 to 6 carbon atoms and 1 to 2 halo groups, alkoxy of from 1 to 6 carbon atoms, -$NR^6R^7$ where $R^6$ and $R^7$ are independently selected from the group consisting of hydrogen and alkyl of from 1 to 6 carbon atoms, and aryl of from 6 to 10 carbon atoms, and -N($CH_3$)O$CH_3$;
X is selected from the group consisting of -O-, -$NR^9$-, and -S- where $R^9$ is selected from the group consisting of hydrogen, alkyl of from 1 to 6 carbon atoms and aryl of from 6 to 10 carbon atoms;
Y is selected from the group consisting of -C(O)- and -C(S)-;
m is equal to zero or one; and
n is equal to zero, one or two,
or pharmaceutically acceptable salts thereof
with the proviso that when $R^1$ is 1-naphthyl, $R^2$ is -CH$(CH_3)_2$ (L-isomer), $R^3$ is -$CH_2$-∅ (L-isomer), Y is -C(O)-, m is zero and n is one, then $R^4$ is not - N($CH_3$)O$CH_3$, with the further proviso that when R' is diphenylmethyl,
$R^2$ is p-(benzyloxy)benzyl (L-isomer), Y is -C(O)-, and m and n are zero, then
$R^4$ is not -N($CH_3$)O$CH_3$, and with still the further proviso that when $R^1$ is (1,2diphenyl)ethenyl, Y is -C(O)-, $R^2$ is -$CH_2$-∅(L-isomer), and m and n are zero, then $R^4$ is not -N($CH_3$)O$CH_3$.

In a preferred embodiment

$R^1$ includes benzyl, trityl, diphenylmethyl, 4phenylbutyl, 2-phenylethyl, naphthyl, pyridyl, fluorenyl, xanthanilyl, and the like.

$R^2$ and $R^3$ are independently side chains of a D- or L- amino acid having at least 2 carbon atoms with the proviso that $R^2$ and $R^3$ are not proline. Such side chains refer to the $R^8$ substituent found on naturally occurring and synthetic amino acids of the formula $H_2NCHR^8COOH$. Side chains of naturally occurring amino acids include, by way of example only, those where $R^8$ is the L-isomer of $(CH_3)^2$CH- (valine), $(CH_3)_2CHCH_2$ (leucine), $CH_3CH_2CH(CH_3)$-(isoleucine), ∅$CH_2$- (phenylalanine), (3-indolyl)-$CH_2$- (tryptophan), $CH_3SCH_2CH_2$- (methionine), $CH_3CH(OH)$ (threonine), p-HO-∅-$CH_2$- (tyrosine), $H_2NC(O)CH_2$- (asparagine), $H_2NC(O)CH_2CH_2$- (glutamine), $HOC(O)CH_2$- (aspartic acid), $HOC(O)CH_2CH_2$- (glutamic acid), $H_2NCH_2CH_2CH_2CH_2$- (lysine), $H_2NC(NH)NHCH_2CH_2CH_2$- (arginine), 4-imidazolyl-$CH_2$- (histidine) and the like.

Side chains of synthetic amino acids include the D-isomer of the above noted naturally occurring amino acids as well as those where $R^8$ is selected from the group consisting of alkyl of from 2 to 6 carbon atoms (where the alkyl group does not occur in naturally occurring amino acids), cycloalkyl of from 3 to 8 carbon atoms, and alkyl of from 1 to 6 carbon atoms substituted with from 1 to 2 substituents selected from the group consisting of
aryl of from 6 to 10 carbon atoms,
aryl of from 6 to 10 carbon atoms substituted with from 1 to 3 substituents selected from the group consisting of alkyl of from 1 to 6 carbon atoms, alkoxy of from 1 to 6 carbon atoms, aryl of from 6 to 10 carbon atoms, and aryloxy of from 6 to 10 carbon atoms, and
heteroaryl of from 3 to 14 carbon atoms having from 1 to 3 heteroatoms selected from the group consisting of nitrogen, oxygen and sulfur (where the substituted alkyl group does not occur in naturally occurring amino acids).
Particularly preferred amino acid side chains include the D- and L-isomers of valine, leucine, phenylalanine, tryptophan and isoleucine.
$R^4$ preferably is -CH=N=N or -C(O)H.
X preferably is -O-.
Y preferably is -C(O)-.
*m* and *n* preferably are 0 or 1.
In the present application -∅ means a phenyl residue.
Preferred compounds include, by way of example, the following compounds as defined by formula II below, including all isomers thereof, wherein the amino acid side chain for $R^2$ and $R^3$ is indicated beneath the $R^2$ and $R^3$ substituent:

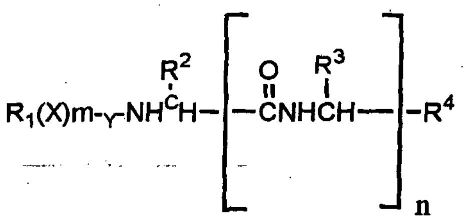

formula II

| R | X | m | Y | $R^2$ | n | $R^3$ | $R^4$ |
|---|---|---|---|---|---|---|---|
| ∅-$CH_2$- | 0 | 1 | -C(O)- | -$CH(CH_3)_2$ (valine) | 1 | -$CH_2$-∅ (phenylalanine) | -C(O)CH=N=N |
| ∅-$CH_2$- | 0 | 1 | -C(O)- | -$CH_2$-∅ (phenylalanine) | 1 | -$CH_2$-∅ (phenylalanine) | -C(O)CH=N=N |
| (∅)$_2$-CH- | - | 0 | -C(O)- | -$CH_2$-∅ (phenylalanine) | 0 | - | -C(O)H |
| ∅-$(CH_2)_4$- | - | 0 | -C(O)- | -$CH_2$-∅ (phenylalanine) | 0 | - | -C(O)H |
| (∅)$_3$-C- | - | 0 | -C(O)- | -$CH_2$-∅ (phenylalanine) | 0 | - | -C(O)H |
| (∅)$_2$-CH- | - | 0 | -C(O)- | -$CH_2$-$CH_2$-∅ (homophenylalanine) | 0 | - | -C(O)H |
| ∅CH=C(∅) | - | 0 | -C(O)- | -$CH_2$-∅ (phenylalanine) | 0 | - | -C(O)H |
| (∅)$_2$-CH- | - | 0 | -C(O)- | -$CH_2$-(3-indolyl) | 0 | - | -C(O)H |
| ∅-$CH_2$- | 0 | 1 | -C(O)- | -$CH_2$-∅ (phenylalanine) | 1 | -$CH(CH_3)_2$ (valine) | -C(O)H |
| ∅-$CH_2$- | 0 | 1 | -C(O)- | -$CH(CH_3)_2$ (valine) | 1 | -$CH_2$-$CH(CH_3)_2$ (leucine) | -C(O)H |

The term "heterocycles containing from 3 to 14 carbon atoms and 1 to 3 hetereoatoms selected from the group consisting of nitrogen, oxygen and sulfur" refers to saturated and unsaturated heterocyclic groups having the requisite number of carbon atoms and heteroatoms. Suitable heterocyclic groups include, by way of example, furazanyl, furyl, imidazolidinyl, imidazolyl, imidazolinyl, indolyl, isothiazolyl, isoxazolyl, morpholinyl (e.g. morpholino), oxazolyl, piperazinyl (e.g. 1-piperazinyl), piperidyl (e.g. 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g. 1-pyrrolidinyl), pyrrolinyl, pyrrolyl, quinoxalinyl, thiadiazolyl, thiazolyl, thienyl, thiomorpholinyl (e.g. thiomorpholino), triazolyl, and xanthanilyl.

If in the present application a chemical group is described as containing for example "from 1 to 6 carbon atoms" or "from 6 to 10 carbon atoms" or "from 1 to 4 heteroatoms" it is meant that the group can contain either 1 or 2 or 3 or 4 or 5 or 6, or 6 or 7 or 8 or 9 or 10, or 1 or 2 or 3 or 4 of said atoms, respectively.

Heterocyclic groups can be substituted or unsubstituted. Where the heterocyclic group is substituted, the substituents are selected from alkyl of from 1 to 6 carbon atoms, alkoxy of from 1 to 6 carbon atoms, aryl of from 6 to 10 carbon atoms, aryloxy of from 6 to 10 carbon atoms, and halo.

Preferred heterocycles include well known cyclic aromatic groups containing heteroatoms within the cyclic structure. Such groups include, by way of example, furyl, imidazolyl, oxazolyl, pyrazolyl, pyridyl, pyrimidinyl, thiazolyl, and triazolyl.

The term "alkyl" refers to straight and branched chain alkyl groups such as methyl, ethyl, n-propyl, iso-propyl, n-butyl, tert-butyl, sec-butyl, isobutyl, n-pentyl, n-hexyl, 2-methylpentyl, and the like; whereas the term "alkoxy" refers to -O-alkyl substituents.

The term "aryl" refers to aromatic substituents comprising carbon and hydrogen such as phenyl, naphthyl and

the like whereas the term aryloxy refers to -O-aryl substituents where aryl is as defined above.

The term "halo" or "halogen" refers to fluorine, chlorine, bromine and iodine and preferably fluorine and chlorine.

The term "pharmaceutically acceptable salts" refers to the non-toxic alkali metal, alkaline earth metal, and ammonium salts commonly used in the pharmaceutical industry including the sodium, potassium, lithium, calcium, magnesium, barium, ammonium, and protamine zinc salts, which are prepared by methods well known in the art. The term also includes non-toxic acid addition salts, which are generally prepared by reacting the compounds of this invention with a suitable organic or inorganic acid.

Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, and napsylate salts, and the like. The particular salt employed is not critical.

Particularly preferred embodiments of the compounds usable as a medicament for the treatment of pain by inhibiting protein activity of Cathepsin Y are the compounds 1 to 13 as shown in the following:

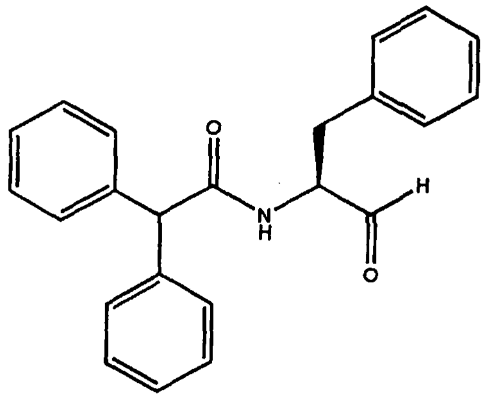

Compound 1

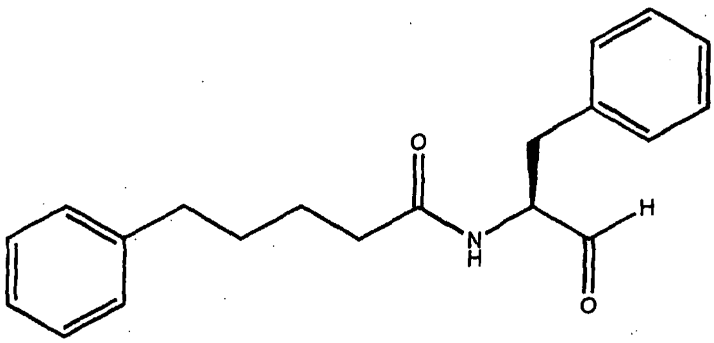

Compound 2

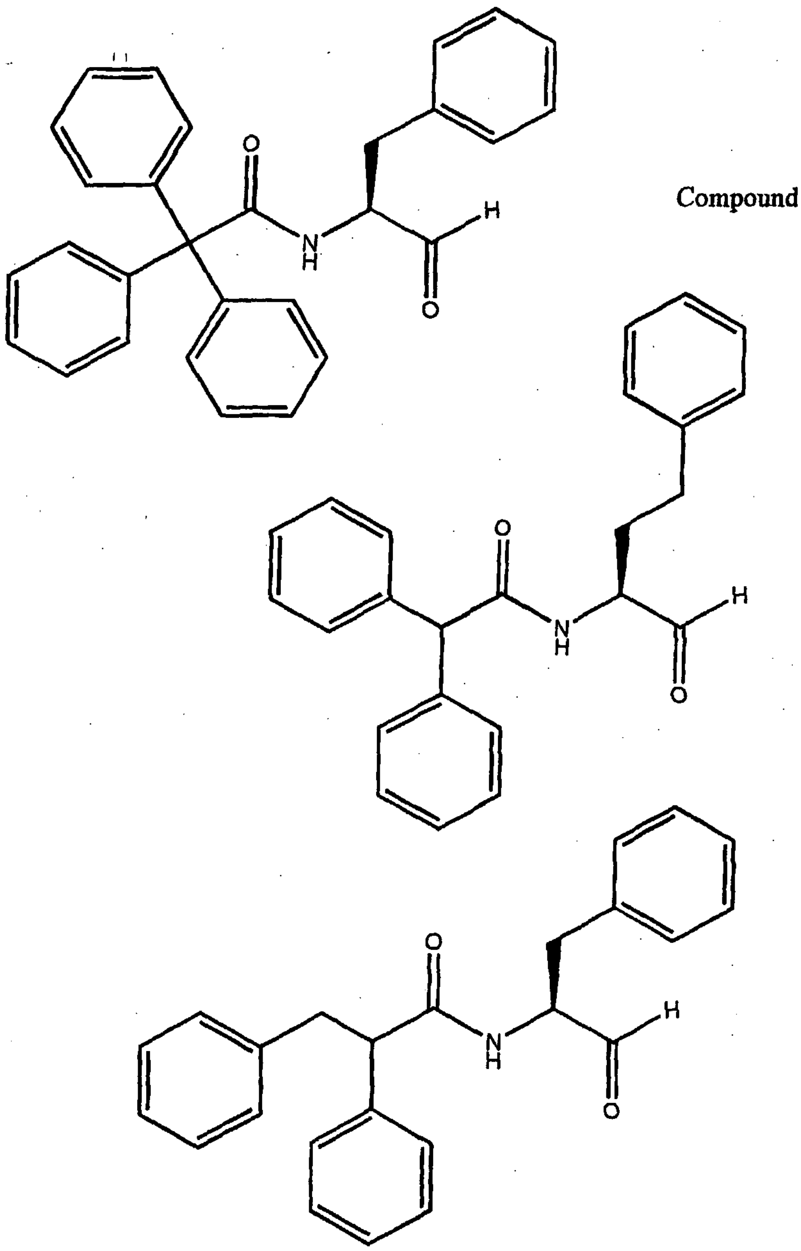

Compound 3

Compound 4

Compound 5

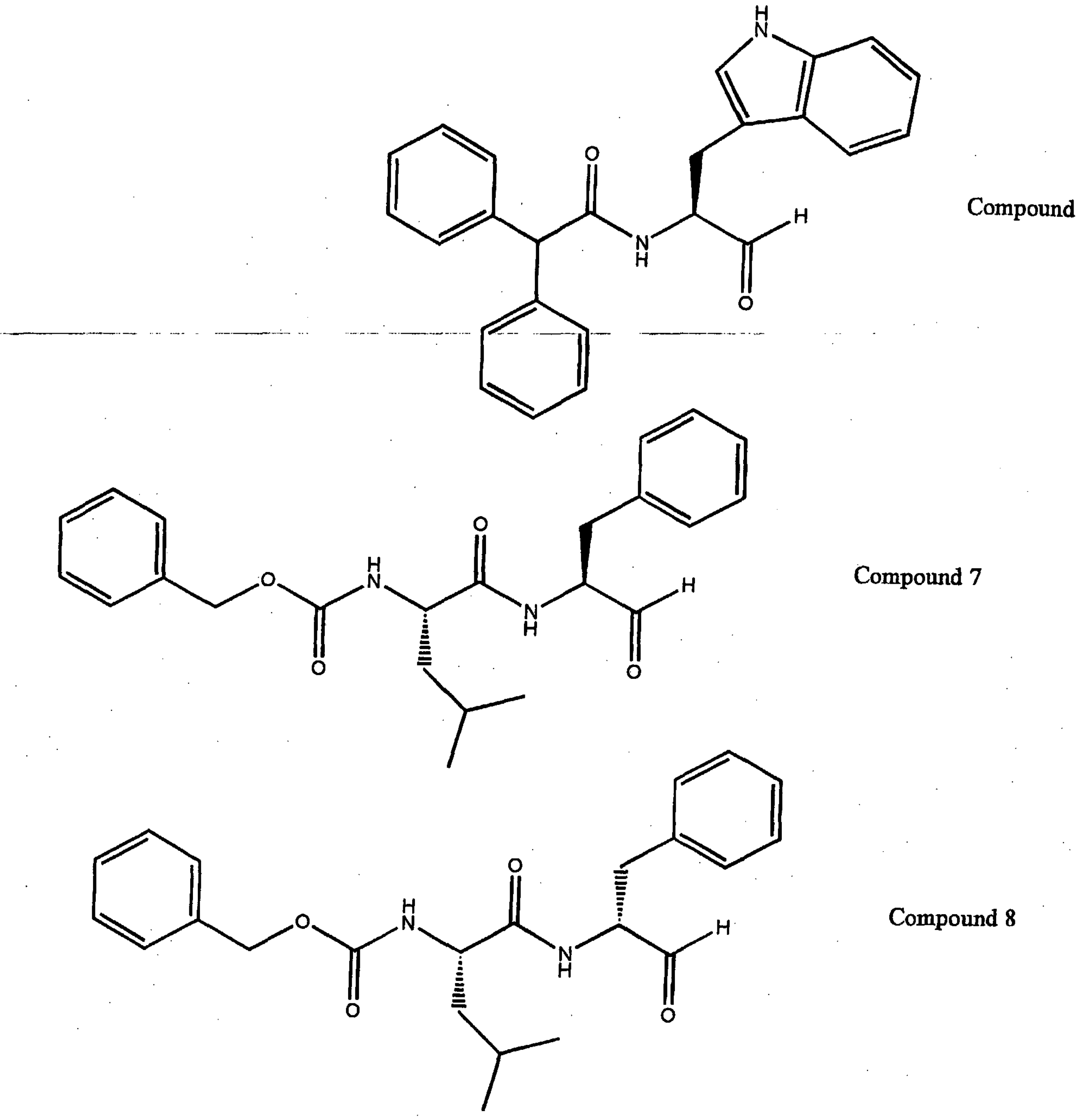

Compound 6

Compound 7

Compound 8

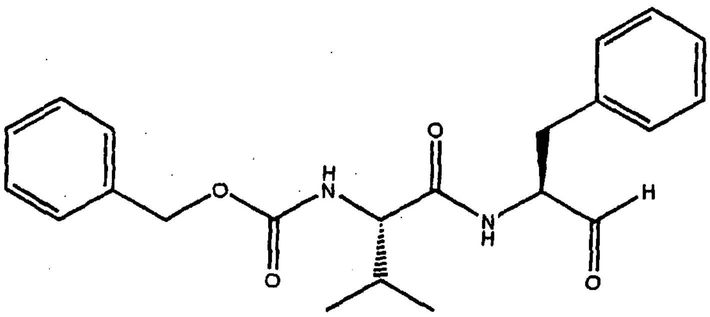

Compound 9

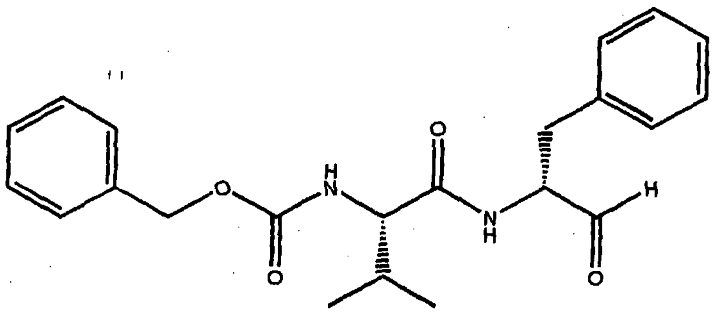

Compound 10

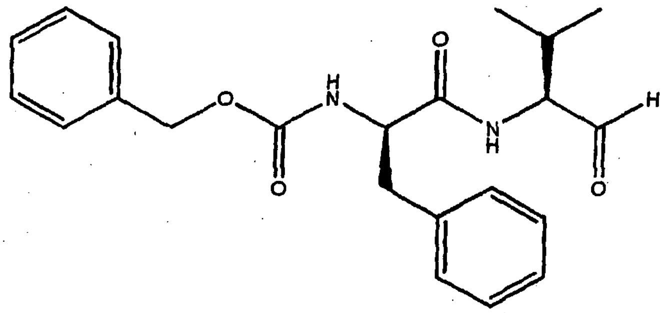

Compound 11

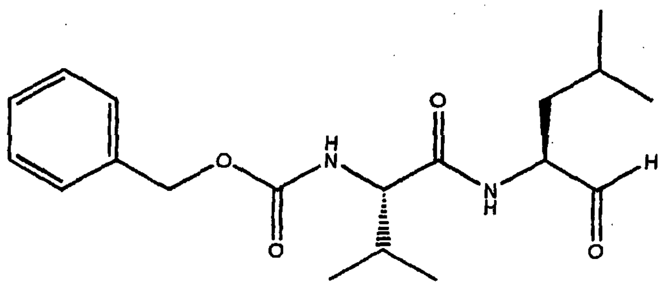

Compound 12

Preparation of the compounds 1 to 12 of the present application is explained in detail in WO96/39194.

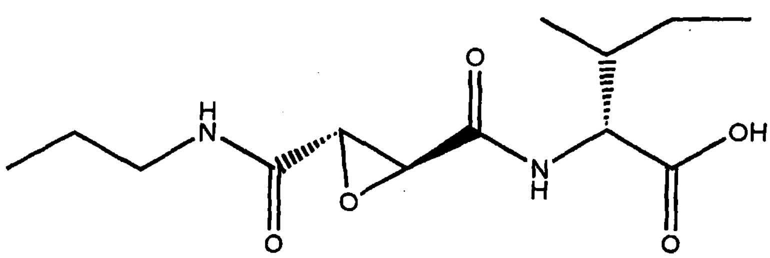

Compound 13

Preparation of compound 13 is explained in detail in the article of Therrien, C. et al., Biochemistry (2001) 40, 2702-2711.

Furthermore the sequences of the present invention itself can be used as a medicament.

Furthermore the sequences of the present invention itself can be used as a medicament.

An example for such a use is the use of a polynucleotide sequence as an antisense agent. Antisense agents can hybridise to DNA or mRNA, inhibiting or decreasing transcription or translation, respectively. Thus, polynucleotide

sequences of a gene which is increased in expression rate under pain can be used as antisense agents to decrease the expression rates of said gene. Further such polynucleotide sequences can be used for gene therapy.

A pharmaceutical composition comprising a polynucleotide sequence according to the present invention can be any composition which can serve as a pharmaceutical one. Salts or aids for stabilising the sequence in the composition preferably are present.

For the determination of the expression of the relevant genes the generated sequences have to be detected. Therefore several reagents can be used, which are for example specific radioactive or non-radioactive (e.g. biotinylated or fluorescent) probes to detect nucleic acid sequences by hybridisation, primer sets for the detection of one or several of the nucleic acid sequences by PCR, DNA microarrays, antibodies against one of the polypeptides, or epitopes or antibody or protein microarrays. Such reagents can be combined in a kit, which can be sold for carrying out any of the described methods.

For further examinations or experiments it might be desirable to include the nucleic acid sequence of the present invention into a vector or a host cell. By including the sequences in a host cell for example cellular assays can be developed, wherein the genes, polynucleotide sequences and the according proteins / polypeptides further can be used or examined.

Further the sequences defined in the present invention can be used to "design" new transgenic animals as models for pain. Therefore the animals are "created" by manipulating the gene(s) encoding Cathepsin Y in a way that their expression in the transgenic animal differs from the expression of the same gene(s) in the wild type. "Manipulation" preferably results in a different expression level of the Cathepsin Y in the transgenic animal compared to the wild type, or in a protein with different enzymatic activity. Whether the expression level or the protein activity becomes higher or lower than the wild type under the same conditions depends from the desired model for pain. Methods of gene manipulation and methods for the preparation of transgenic animals are commonly known to those skilled in the art.

Figures:

Figure 1 shows comparison experiments of three pain models CCI, PSL and SNL. Expression levels of Cathepsin Y gene are compared over a time period of 28 days to sham operated controls. Cathepsin Y is described as differentially expressed when the sequence is up or down regulated at one time point in at least two of the three models. Over time the expression pattern can be determined as up-regulated in one to four time points; as down regulated in one to four time points or as mixed regulated if the type of regulation changes between up and down regulation at different time points.

X-axis describes the four time points analysed by DEPD, 1= day one post operation, 2= day 7 post operation, 3= day 14 post operation, 4=day 28 post operation. The Y-axis shows Δ h which represents the normalised difference of expression (peak height) of a certain transcript between a control group and a treated group. x-fold difference in gene expression is calculated by

$$\frac{1+\Delta h}{1-\Delta h}$$

0= no change to control, + = up regulation, - = down regulation; (0,2 = 1,5 fold; 0,3 = 1,86 fold; 0,4 = 2,33 fold; 0,5 = 3 fold).

The following examples are provided for illustration and are not intended to limit the invention to the specific example provided.

Examples:

Example 1: Preparation of rat models

A) The CCI model: (Bennett and Xie , Pain 1988, 33:87-107)

Nerve injury is created by loosely constrictive ligatures around rat sciatic nerve (4 ligatures). The ligatures evoke intraneural edema, the swelling is opposed by the ligatures of the nerve strangulates. The constrictions remain for at least a month (hence "chronic constriction injury" CCI). It is known that the constriction injures nearly all of the nerves large myelinated axons; however, a variable but large percentage of the nerves unmyelinated axons remained intact. Although the nerve distal to the constriction is full of degeneration, the nerve proximal to the constriction appears

normal, and there is no evidence of any primary afferent neuron dying. This model has a periphery that is innervated only by C-fibers and a greatly reduced number of A-delta fibers, and a spinal cord that is innervated by injured (but alive) A-beta low-threshold mechanoreceptors (ABLTMs), A-delta fibers (mostly injured, a few intact) and both injured and intact C-fiber afferents, many of which are nociceptors. This animal model provokes allodynia and hyperalgesia as well as spontaneous pain. Evidence of abnormal pain sensation is detected in the majority of CCI cases on the second PO day, and in nearly all cases by 5-7 days post injury. Abnormal pain sensation appears to reach peak severity in 10-14 days and to disappear in about 2 month when the pain is replaced by an apparently permanent state of hyperaesthesia.

In detail, male Lewis rats (150-350g body weight) were anaesthetised with sodium pentobartital (50mg/kg i.p.). Thereafter the common sciatic nerve on the left side was exposed at the level of the middle of the thigh by blunt dissection through the biceps femoris. Proximal to the sciatic trifurcation, about 7 mm of nerve was freed from adhering tissues and 4 ligatures (4.0 chromic gut) were tied loosely around it with 1 mm spacing. The length of nerve affected was 4-5 mm long. The desired degree of constriction retarded, but did not arrest, circulation through the superficial epineurial vasculature and sometimes produced a small, brief twitch in the muscle surrounding exposure. Control group animals were sham ligation (only left site), which represents the same operative procedure as the ligated one but without nerve ligation.

Four time points for gene expression profiling were chosen: 1day, 7 days, 14 days, and 28 days post operation. Animals were tested for mechanical allodynia (von Frey test) one day before surgery and within 30-60 min before tissue preparation (dorsal root ganglia, spinal cord and thalamus). Tissues were frozen on liquid nitrogen prior to RNA preparation.

B) The PSL model: Seltzer, Dubner, and Shir (Pain 1990, 43: 205-218)

Disorders in nocifensive behavior following noxious and non-noxious stimuli begin hours after partial sciatic nerve injury and last for at 7 month.

This model is characterized by complex combination of rapid onset, allodynia to touch, hyperalgesia, mirror image phenomena, and dependence on the sympathetic outflow. This model resembles therefore many of the symptoms described for causalgia in man. The rapid initiation of these disorders and their contralateral appearance suggest central reorganization. This model may serve as a model for sympathetically maintained pain (SMP)

In detail Lewis rats (male 150-350g body weight) were anaesthetised with sodium pentobartital (50mg/kg i.p.) followed by exposure of the left sciatic nerve at high-thigh level. Under 25x magnification, the dorsum of the nerve was carefully freed from surrounding tissues at a site near the trochanter just distal to the point at which the posterior biceps semitendinous ('PBST') nerve branches off the common sciatic nerve. An 8-0 silicon-treated silk suture was inserted into the nerve with a 3/8 curved, reversed-cutting mini-needle, and tightly ligated so that the dorsal 1/3 to ½ of the nerve thickness was trapped in the ligature.

Control group animals were sham ligated, which represents the same operative procedure as the ligated one but without nerve ligation.

Four time points for gene expression profiling were chosen: 1day, 7 days, 14 days, and 28 days post operation. Animals were tested for mechanical allodynia (von Frey test) one day before surgery and 30-60 min before tissue preparation (dorsal root ganglia, spinal cord and thalamus). Tissues were frozen on liquid nitrogen prior to RNA preparation.

C) The SNL model (Kim and Chung Pain 50 (1992) 355-363)

In this model a tight ligation of the left L5 and L6 spinal nerves is leading to a long-lasting hyperalgesia to noxious heat, at least 5 weeks of the affected foot. Long-lasting mechanical allodynia of the affected foot can be observed for at least 10 weeks. This model involves a complete ligation of spinal nerves L5 and L6 or only L5 (or L4), which is more reliable to other models where the number and types of ligated nerves are difficult to control

In detail, male Lewis rats (150-350g body weight) were anaesthetised with sodium pentobartital (50mg/kg i.p.). Thereafter the sciatic spinal nerve ligation of the spinal nerve L5 was performed on the left site of the animals.

Control group animals were sham ligation (only left site), which represents the same operative procedure as the ligated ones but without nerve ligation.

Four time points for gene expression profiling were chosen: 1day, 7 days, 14 days, and 28 days post operation. Animals were tested for mechanical allodynia (von Frey test) one day before surgery and 30-60 min before tissue preparation (dorsal root ganglia, spinal cord and thalamus). Tissues were frozen on liquid nitrogen prior to RNA preparation.

Example 2: Determination of expression levels

Gene expression profiling by DEPD-analysis starts with the isolation of 5-10μg total RNA. In a second step, double-stranded cDNA is synthesized. Through an enzymatic digest of the cDNA with three different type IIS restriction enzymes, three pools with short DNA-fragments containing single-stranded overhangs are generated. Afterwards, specific DNA-adaptor-molecules are ligated and in two subsequent steps 3.072 PCR reactions are performed by using 1024 different unlabelled 5'primer and a common FAM fluorescent labelled 3'-primer in the last PCR step. Subsequently, the 3072 PCR pools are analysed on an automatic capillary electrophoresis sequencer. Differential gene expression pattern of single fragments are determined by comparison of normalized chromatogram peaks from the control groups and

corresponding operated animals.

Example 3: Sequencing and Databank analysis of the obtained sequences

Differentially expressed peaks are confirmed on polyacrylamid gels by using radioactive labelled 3' primer instead of the FAM fluorescent primer. Differentially expressed bands are cut from the gel. After a short elution step in 60μl 10mM Tris pH8, fragments are re-amplified by PCR using the same primer as used in the DEPD analysis. Resulting PCR products are treated with a mixture of Exonuclease I and shrimp alkaline phosphatase prior to direct sequencing. Sequencing reactions are performed by using a sequencing kit like DYE-namic ET dye terminator sequencing kit, and subsequently analysed by capillary electrophoresis (Megabace 1000, Amersham).

Prior to a BLAST analysis (Altschul et al. 1997, Nucleic Acids Res. 25:3389-3402) against Genbank and Unigene, sequences are quality verified and redundant sequences or repetitive motifs are masked.
Results are shown in Table 1 and are compared to human sequence.

Expression of the Cathepsin Y gene over a time period of 28 days after preparation of the pain model is shown in figure 1.

| SEQ ID NO: | name | length |
|---|---|---|
| 1 | EST of Cathepsin Y (rat) | 255 bp |
| 2 | rattus norvegicus Cathepsin Y DNA | 1387 bp |
| 3 | rat Cathepsin Y protein | 306 aa |
| 4 | human Cathepsin Y DNA | 1500 bp |
| 5 | human Cathepsin Y protein | 303 aa |

Example 4: Enzymatic activity of Cathepsin Y protein

Cathepsin Y does not have the standard endopeptidic activity associated with proteases. In an effort to identify whether the enzyme has other proteolytic activity, a number of randomly selected synthetic oligopeptides were incubated with purified Cathepsin Y at pH 5.5 or 4.5. Specifically, purified Cathepsin Y was incubated with the selected synthetic oligopeptides (50 μg/ml) at either pH 4.5 or pH 5.5, for 1 hour at 37°C.

Samples were quenched by the addition of trifluoroacetic acid to 1% final concentration, then analysed by reverse phase HPLC on a Vydac C18 column, using a gradient of increasing acetonitrile in 0.1% trifluoroacetic acid. Individual peaks (parent and new product(s)) were collected, then analysed by acid hydrolysis followed by amino-acid analysis.

The results are summarised below:

| Parent Sequence | Product(s) |
|---|---|
| LFYDQSPTATI | LFYDQSPTAT(aa 1-10 of the parent sequence)<br>LFYDQSPTA (aa 1-9 of the parent sequence)<br>LFYDQSPT (aa 1-8 of the parent sequence) |
| YKRDMVGGVVIA | YKRDMVGGVVI (aa 1-11 of the parent sequence)<br>YKRDMVGGVV (aa 1-10 of the parent sequence) |
| EGYYGNYGVYA | EGYYGNYGVY (aa 1-10 of the parent sequence)<br>EGYYGNYGV (aa 1-9 of the parent sequence) |
| FFDEPNPGUTIY | FFDEPNPGVT (aa 1-10 of the parent sequence) |

The above peptides, as well as other peptides recited herein, are listed, per convention, from amino terminus to the carboxyl terminus.

The results from a number of such experiments (all not shown) suggest that the proteolytic activity of Cathepsin Y is a sequential removal of the carboxy terminal amino-acids which is direct evidence for carboxypeptidase activity. No evidence of any endopeptidase or aminopeptidase activity was seen with any of these substrates. These data strongly suggest that the predominant proteolytic activity manifested by Cathepsin Y is carboxypeptidase activity.

The quantitative analysis of carboxypeptidase activity of Cathepsin Y is based on the detection of the new free

amino-terminus generated on cleavage of a selected substrate. This is accomplished by reacting with the reagent ophthalaldehyde in an alkaline solution in the presence of 2-mercaptoethanol (Simons, et al., JACS, 98:7098-7099, (1976)). The peptide EGYYGNYGV was synthesised acetylated on its amino-terminus so that on cleavage by Cathepsin Y, the only free amino-terminus present in the reaction mixture will be that of the valine residue, cleaved off by the carboxypeptidase activity of the protease.

A standard curve was constructed by incubating varying concentrations of valine (0-20 μM) in 0.25 M sodium borate, pH 10, containing 0.05% 2-mercaptoethanol and 60 μg/ml o-phthalaldehyde, in individual wells of a 96 well microtiter plate. The resulting fluorescence is read in a plate reading Cytofluor (ex 340, em 460 nm). There is a linear increase in the signal proportional to the amount of free valine present

In order to determine the pH optimum of the enzyme, reaction mixtures were set up with enzyme and substrate (0.2 mg/ml) in a total reaction volume of 0.1 ml) in individual wells of 96-well microtiter plates, in 20 mM sodium acetate buffers at different pHs, with 0.1 % 2-mercaptoethanol present. Control wells were set identically except for the presence of enzyme. At various timepoints after incubation at room temperature, the reactions were quenched by the addition of an equal volume of 0.45 M sodium borate, pH 10, with 0.25 mg/ml o-phthalaldehyde, and the fluorescence measured. In the absence of added enzyme, no measurable fluorescence is generated above background, even with extended incubations. In the presence of enzyme, there is a time-dependent increase in fluorescence. Based on this analysis, the pH optimum for carboxypeptidase activity was determined to be about 4.5, with the activity dropping off at both lower and higher pHs.

Using this assay at pH 4.5, the ability of a number of compounds to inhibit the activity of Cathepsin Y was tested, by adding the desired concentration of the inhibitor in the incubation mixture along with enzyme and substrate, and measuring the decrease in fluorescence (if any) relative to enzyme control. Each of the compounds 1 to 13 tested in this analysis indicated inhibition of Cathepsin Y activity.

## Claims

1. Method for the development of a medicament for the treatment of pain comprising the use of a polynucleotide sequence selected from SEQ ID NO:1, 2 or 4 encoding Cathepsin Y or homologues or the according protein / polypeptide, comprising determination of the efficiency of a compound or agent in a model for pain, whereby the efficiency of the compound / agent is determined by considering expression rate of the gene or by considering activity of the protein.

2. Method according to claim 1, comprising

   a) providing a test cell population comprising cells capable of expressing the Cathepsin Y gene homologues or fragments thereof
   b) contacting said test cell population with the test therapeutic agent;
   c) detecting the expression of the Cathepsin Y gene(s) in said test cell population,
   d) comparing the expression of the gene(s) in the test cell population to the expression of the gene(s) in a reference cell population whose disease stage is known; and
   e) identifying a difference in expression levels of the considered sequences, if present, in the test cell population and the reference cell population, thereby identifying a therapeutic agent for treating pain.

3. Method according to claims 1 or 2, comprising

   a) contacting the Cathepsin Y protein with a compound /agent in presence of a polypeptide which is a target of the Cathepsin Y,
   b) determining the aminoterminal amino acid of the peptide or the amount of free amino acids in the sample after incubation,
   c) comparing the aminoterminal amino acid of the peptide or the amount of free amino acids with the result in a sample which doesn’t contain the compound / agent.

4. Method according to claim 2 or 3, whereby the gene expression is considered or determined by PCR of cDNA, hybridisation of a sample DNA or by detecting the according protein.

5. Use of a compound downregulating activity of Cathepsin Y encoded by SEQ ID NO:1, 2 or 4 for the manufacture of a medicament for the treatment of pain, whereby the compound has the general formulae

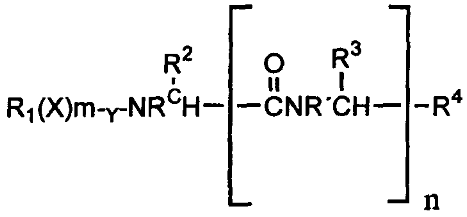

formula I

wherein:

R is selected from the group consisting of hydrogen, alkyl of from 1 to 6 carbon atoms, and where R and $R^2$ are joined to form a ring structure of from 4 to 10 carbon atoms,
R' is selected from the group consisting hydrogen, alkyl of from 1 to 6 carbon atoms and where R' and $R^3$ are joined to form a ring structure of from 4 to 10 carbon atoms,
$R^1$ is selected from the group consisting of
alkyl of from 1 to 4 carbon atoms substituted with from 1 to 5 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur
wherein said substituted alkyl group is optionally further substituted with from 1 to 2 hydroxyl groups,
alkenyl of from 2 to 4 carbon atoms substituted with from 1 to 4 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur,
aryl of from 6 to 10 carbon atoms,
aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, fluorenyl,
heterocycles of from 3 to 14 carbon atoms having from 1 to 3 heteroatoms selected from the group consisting of nitrogen, oxygen and sulfur;
$R^2$ and $R^3$ are independently D- or L-amino acid side chains of at least 2 carbon atoms with the proviso that said amino acid side chains do not include the proline side chain;
$R^4$ is selected from the group consisting of
$-C(O)CH=N=N$, $-CH_2OH$, $-C=NOH$, and $-C(O)R^5$
where $R^5$ is hydrogen, alkyl of from 1 to 6 carbon atoms, haloalkyl of from 1 to 6 carbon atoms and 1 to 2 halo groups, alkoxy of from 1 to 6 carbon atoms, $-NR^6R^7$ where $R^6$ and $R^7$ are independently selected from the group consisting of hydrogen and alkyl of from 1 to 6 carbon atoms, and aryl of from 6 to 10 carbon atoms, and $-N(CH_3)OCH_3$;
X is selected from the group consisting of -O-, $-NR^9-$, and -S- where $R^9$ is selected from the group consisting of hydrogen, alkyl of from 1 to 6 carbon atoms and aryl of from 6 to 10 carbon atoms;
Y is selected from the group consisting of -C(O)- and -C(S)-;
m is equal to zero or one; and
n is equal to zero, one or two,
or pharmaceutically acceptable salts thereof
with the proviso that when $R^1$ is 1-naphthyl, $R^2$ is $-CH(CH_3)_2$ (L-isomer), $R^3$ is $-CH_2-\varnothing$ (L-isomer), Y is -C(O)-, m is zero and n is one, then $R^4$ is not $-N(CH_3)OCH_3$, with the further proviso that when R' is diphenylmethyl, $R^2$ is p-(benzyloxy)benzyl (L-isomer), Y is -C(O)-, and m and n are zero, then
$R^4$ is not $-N(CH_3)OCH_3$, and with still the further proviso that when $R^1$ is (1,2diphenyl)ethenyl, Y is -C(O)-, $R^2$ is $-CH_2-\varnothing$ (L-isomer), and m and n are zero, then $R^4$ is not $-N(CH_3)OCH_3$.

**6.** Use according to claim 5, whereby the compound is selected from the group of

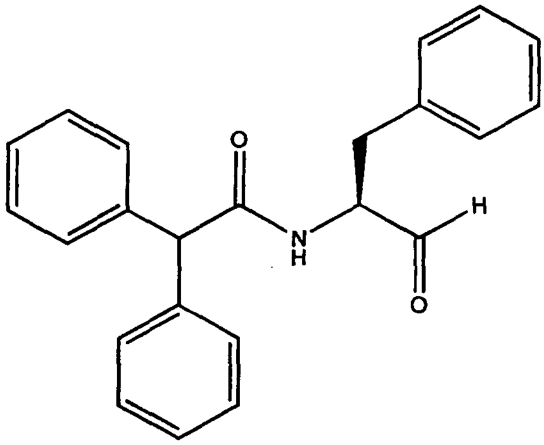

Compound 1

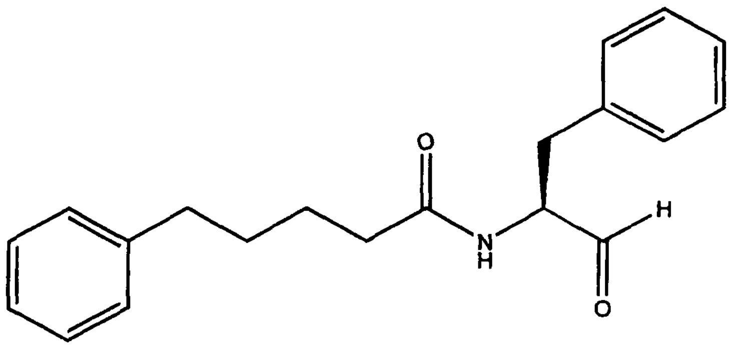

Compound 2

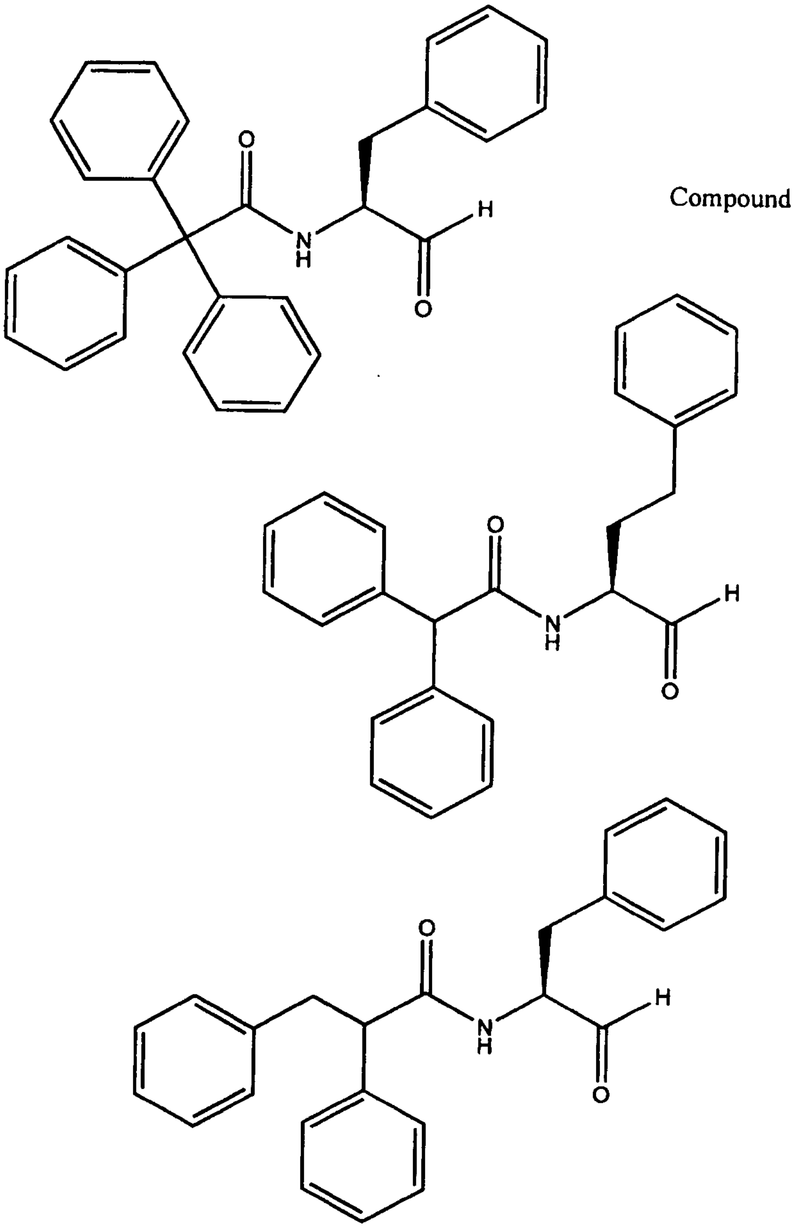

Compound 3

Compound 4

Compound 5

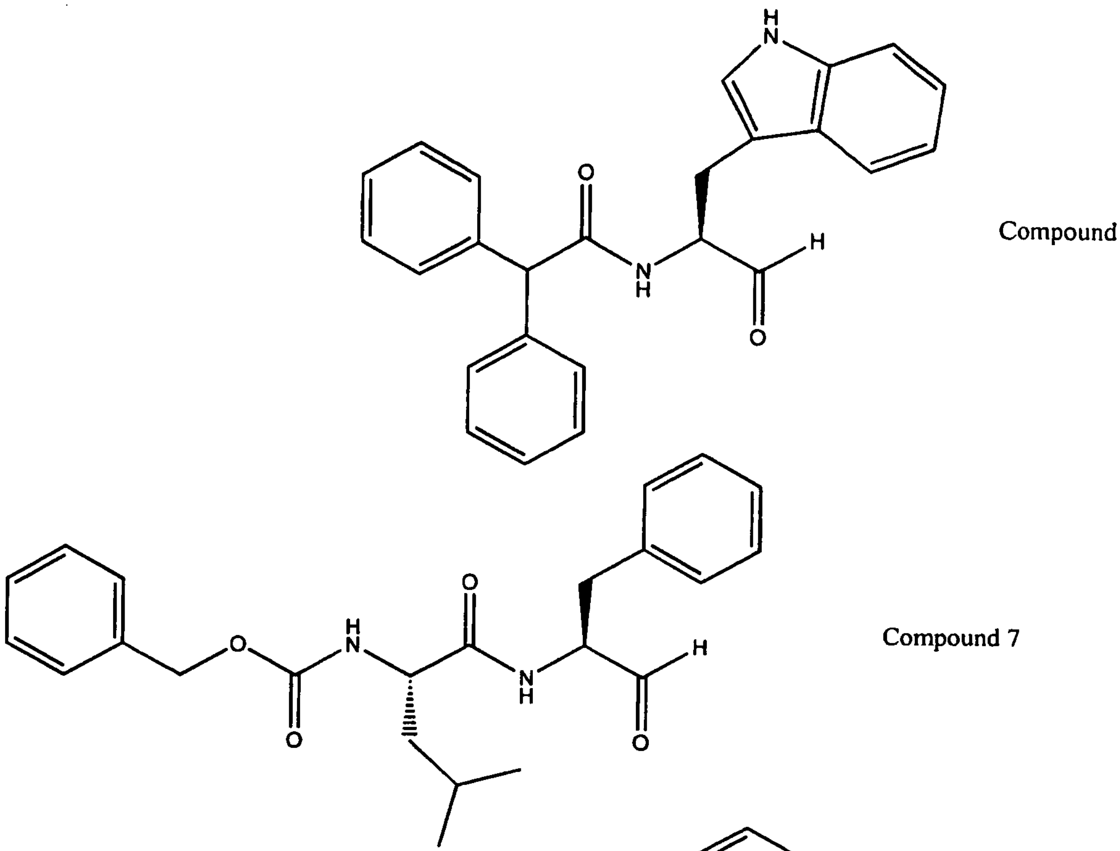

Compound 6

Compound 7

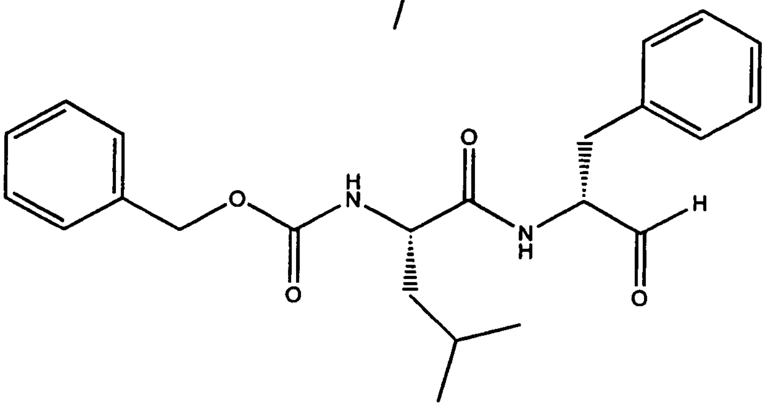

Compound 8

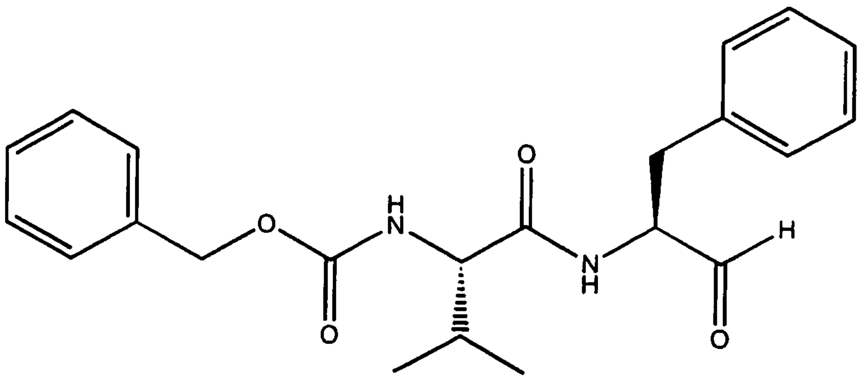

Compound 9

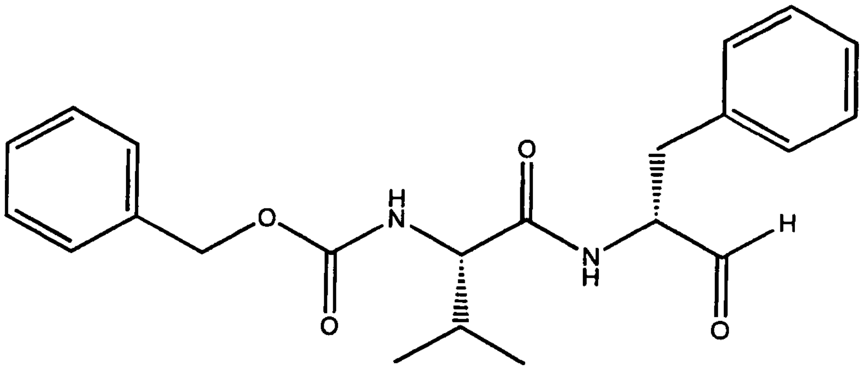

Compound 10

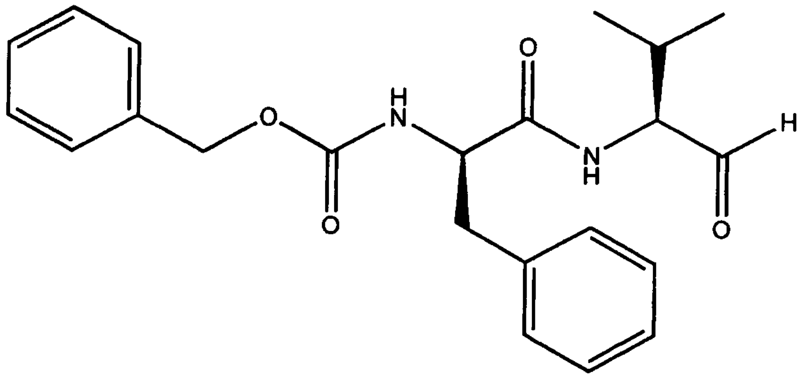

Compound 11

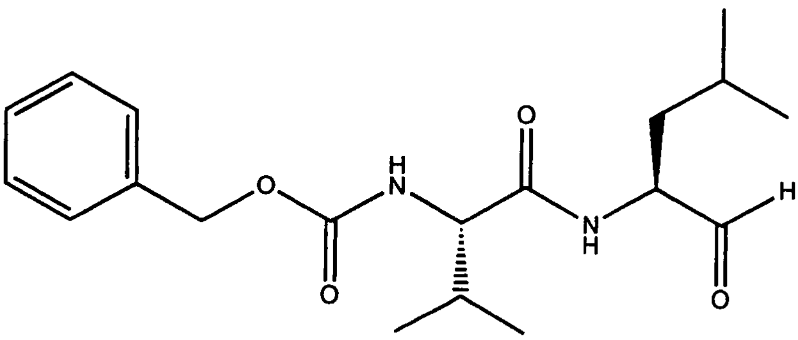

Compound 12

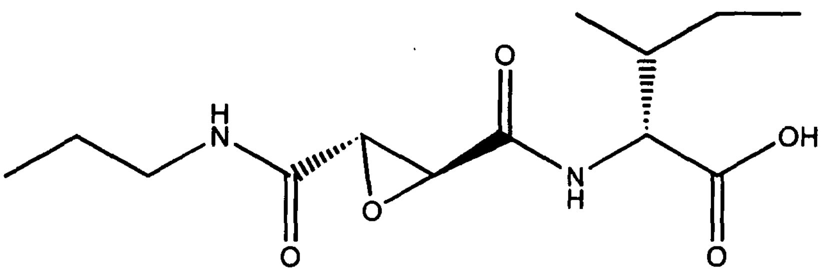

Compound 13

**7.** Method for diagnosing a pain status outside of a living body or for assessing the efficacy of pain treatment outside

of a living body **characterised by** the use of a polynucleotide sequence encoding Cathepsin Y selected from SEQ ID NO: 1, 2 or 4 or homologues the according polypeptide, comprising determination of the efficiency of a compound or agent by considering expression rate of the gene or by considering activity of the protein.

**8.** Method according to any of claims 1 to 4 and 7 or use according to claim 5 or 6 whereby the pain is neuropathic pain.

**9.** A non-human transgenic animal wherein the gene encoding Cathepsin Y according to SEQ ID NO: 1, 2 or 4 or homologues is manipulated in the animal, resulting in different expression level or protein activity in comparison to the wild type.

**Patentansprüche**

**1.** Verfahren zur Entwicklung eines Medikamentes für die Behandlung von Schmerz, umfassend die Verwendung einer Polynucleotidsequenz, ausgewählt aus SEQ ID NO:1, 2 oder 4, welche Cathepsin Y oder Homologe davon kodiert, oder des entsprechenden Proteins / Polypeptids, umfassend das Bestimmen der Wirksamkeit einer Verbindung oder eines Mittels in einem Modell für Schmerz, wobei die Wirksamkeit der Verbindung / des Mittels bestimmt wird, indem die Expressionsrate des Gens oder die Aktivität des Proteins betrachtet wird.

**2.** Verfahren nach Anspruch 1, umfassend

a) Bereitstellen einer Testzellpopulation, enthaltend Zellen, die in der Lage sind, das Cathepsin Y Genhomologe oder Fragmente davon zu exprimieren,
b) In-Kontakt-Bringen dieser Testzellpopulation mit dem therapeutischen Testmittel,
c) Nachweisen der Expression des/der Cathepsin Y Gen(e) in dieser Testzellpopulation,
d) Vergleichen der Expression des/der Gen(e) in der Testzellpopulation mit der Expression des/der Gen(e) in einer Referenzzellpopulation, deren Krankheitszustand bekannt ist, und
e) Identifizieren eines Unterschieds der Expressionsgrade der betrachteten Sequenzen, wenn er vorliegt, in der Testzellpopulation und der Referenzzellpopulation, **dadurch** Identifizieren eines therapeutischen Mittels für die Behandlung von Schmerz.

**3.** Verfahren nach Anspruch 1 oder 2, umfassend

a) In-Kontakt-Bringen des Cathepsin Y Proteins mit einer Verbindung / einem Mittel in Anwesenheit eines Polypeptids, welches ein Target für das Cathepsin Y ist,
b) Bestimmen der aminoterminalen Aminosäure des Peptids oder der Menge an freien Aminosäuren in der Probe nach der Inkubation,
c) Vergleichen der aminoterminalen Aminosäure des Peptids oder der Menge der freien Aminosäure mit dem Ergebnis in einer Probe, welche die Verbindung / das Mittel nicht enthält.

**4.** Verfahren nach Anspruch 2 oder 3, wobei die Genexpression durch PCR von cDNA, durch Hybridisieren einer Proben-DNA oder durch Nachweisen des entsprechenden Proteins betrachtet oder bestimmt wird.

**5.** Verwendung einer Verbindung, die die Aktivität von Cathepsin Y, das durch SEQ ID NO:1, 2 oder 4 kodiert ist, herunterreguliert, zur Herstellung eines Medikamentes für die Behandlung von Schmerz, wobei die Verbindung die allgemeine Formel

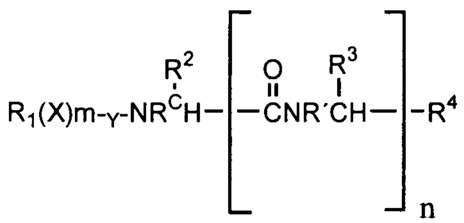

Formel 1

hat, worin:

R ausgewählt ist aus der Gruppe bestehend aus Wasserstoff, Alkyl mit 1 bis 6 Kohlenstoffatomen oder R und $R^2$ verbunden sind, um eine Ringstruktur von 4 bis 10 Kohlenstoffatomen zu bilden,
R' ausgewählt ist aus der Gruppe bestehend aus Wasserstoff, Alkyl mit 1 bis 6 Kohlenstoffatomen oder R' und $R^3$ verbunden sind, um eine Ringstruktur von 4 bis 10 Kohlenstoffatomen zu bilden,
$R^1$ ausgewählt ist aus der Gruppe bestehend aus Alkyl mit 1 bis 4 Kohlenstoffatomen, substituiert mit 1 bis 5 Substituenten, ausgewählt aus der Gruppe bestehend aus (a) Aryl mit 6 bis 10 Kohlenstoffatomen, (b) Aryl mit 6 bis 10 Kohlenstoffatomen, substituiert mit 1 bis 3 Substituenten, ausgewählt aus der Gruppe bestehend aus Alkyl mit 1 bis 6 Kohlenstoffatomen, Aryl mit 6 bis 10 Kohlenstoffatomen, Alkoxy mit 1 bis 6 Kohlenstoffatomen, Aryloxy mit 6 bis 10 Kohlenstoffatomen, Hydroxy, Cyano, Halogen und Amino, (c) Cycloalkyl mit 3 bis 8 Kohlenstoffatomen und (d) Heterocyclen mit 3 bis 14 Kohlenstoffatomen mit 1 bis 3 Heteroatomen, ausgewählt aus der Gruppe bestehend aus Stickstoff, Sauerstoff und Schwefel,
wobei diese substituierte Alkylgruppe optional außerdem mit 1 bis 2 Hydroxylgruppen substituiert ist,
Alkenyl mit 2 bis 4 Kohlenstoffatomen, substituiert mit 1 bis 4 Substituenten, ausgewählt aus der Gruppe bestehend aus (a) Aryl mit 6 bis 10 Kohlenstoffatomen, (b) Aryl mit 6 bis 10 Kohlenstoffatomen, substituiert mit 1 bis 3 Substituenten, ausgewählt aus der Gruppe bestehend aus Alkyl mit 1 bis 6 Kohlenstoffatomen, Aryl mit 6 bis 10 Kohlenstoffatomen, Alkoxy mit 1 bis 6 Kohlenstoffatomen, Aryloxy mit 6 bis 10 Kohlenstoffatomen, Hydroxy, Cyano, Halogen und Amino, (c) Cycloalkyl mit 3 bis 8 Kohlenstoffatomen und (d) Heterocyclen mit 3 bis 14 Kohlenstoffatomen mit 1 bis 3 Heteroatomen, ausgewählt aus der Gruppe bestehend aus Stickstoff, Sauerstoff und Schwefel,
Aryl mit 6 bis 10 Kohlenstoffatomen,
Aryl mit 6 bis 10 Kohlenstoffatomen, substituiert mit 1 bis 3 Substituenten, ausgewählt aus der Gruppe bestehend aus Alkyl mit 1 bis 6 Kohlenstoffatomen, Aryl mit 6 bis 10 Kohlenstoffatomen, Alkoxy mit 1 bis 6 Kohlenstoffatomen, Aryloxy mit 6 bis 10 Kohlenstoffatomen, Hydroxy, Cyano, Halogen und Amino,
Fluorenyl,
Heterocyclen mit 3 bis 14 Kohlenstoffatomen mit 1 bis 3 Heteroatomen, ausgewählt aus der Gruppe bestehend aus Stickstoff, Sauerstoff und Schwefel,
$R^2$ und $R^3$ unabhängig voneinander D- oder L-Aminosäureseitenketten mit wenigstens 2 Kohlenstoffatomen sind, unter der Voraussetzung, dass diese Aminosäureseitenketten keine Prolinseitenkette enthalten;
$R^4$ ausgewählt ist aus der Gruppe bestehend aus
$-C(O)CH=N=N$, $-CH_2OH$, $-C=NOH$ und $-C(O)R^5$,
worin $R^5$ Wasserstoff, Alkyl mit 1 bis 6 Kohlenstoffatomen, Halogenalkyl mit 1 bis 6 Kohlenstoffatomen und 1 bis 2 Halogengruppen, Alkoxy mit 1 bis 6 Kohlenstoffatomen, $-NR^6R^7$, worin $R^6$ und $R^7$ unabhängig voneinander ausgewählt sind aus der Gruppe bestehend aus Wasserstoff und Alkyl mit 1 bis 6 Kohlenstoffatomen und Aryl mit 6 bis 10 Kohlenstoffatomen, und- $N(CH_3)OCH_3$;
X ausgewählt ist aus der Gruppe bestehend aus -O-, $-NR^9-$ und -S-, worin $R^9$ ausgewählt ist aus der Gruppe bestehend aus Wasserstoff, Alkyl mit 1 bis 6 Kohlenstoffatomen und Aryl mit 6 bis 10 Kohlenstoffatomen;
Y ausgewählt ist aus der Gruppe bestehend aus -C(O)- und -C(S)-;
m gleich null oder eins ist und
n gleich null, eins oder zwei ist,
oder pharmazeutisch annehmbare Salze davon
unter der Voraussetzung, dass, wenn $R^1$ gleich I-Naphthyl ist, $R^2$ gleich $-CH(CH_3)_2$ (L-Isomer) ist, $R^3$ gleich $-CH_2-\varnothing$ (L-Isomer) ist, Y gleich -C(O)-ist, m gleich null ist und n gleich eins ist, dann $R^4$ nicht $-N(CH_3)OCH_3$ ist, unter der weiteren Voraussetzung, dass, wenn R' gleich Diphenylmethyl ist, $R^2$ gleich p-(Benzyloxy)benzyl (L-Isomer) ist, Y gleich -C(O)- ist und m und n gleich null sind, dann $R^4$ nicht $-N(CH_3)OCH_3$ ist, und unter der weiteren Voraussetzung, dass, wenn $R^1$ gleich (1,2-Diphenyl)ethenyl ist, Y gleich -C(O)- ist, $R^2$ gleich $-CH_2-\varnothing$ (L-Isomer) ist und m und n gleich null sind, dann $R^4$ nicht $-N(CH_3)OCH_3$ ist.

**6.** Verwendung nach Anspruch 5, wobei die Verbindung ausgewählt ist aus der Gruppe aus

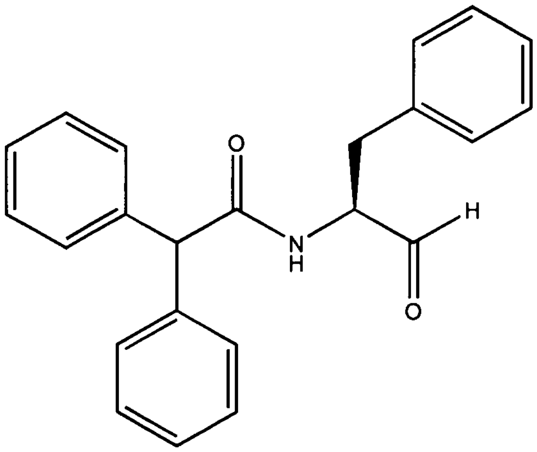

Verbindung 1

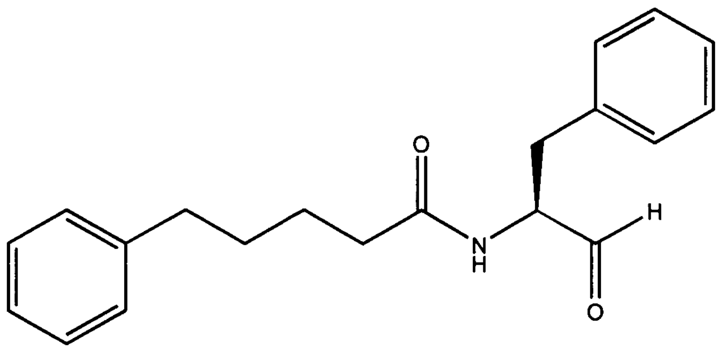

Verbindung 2

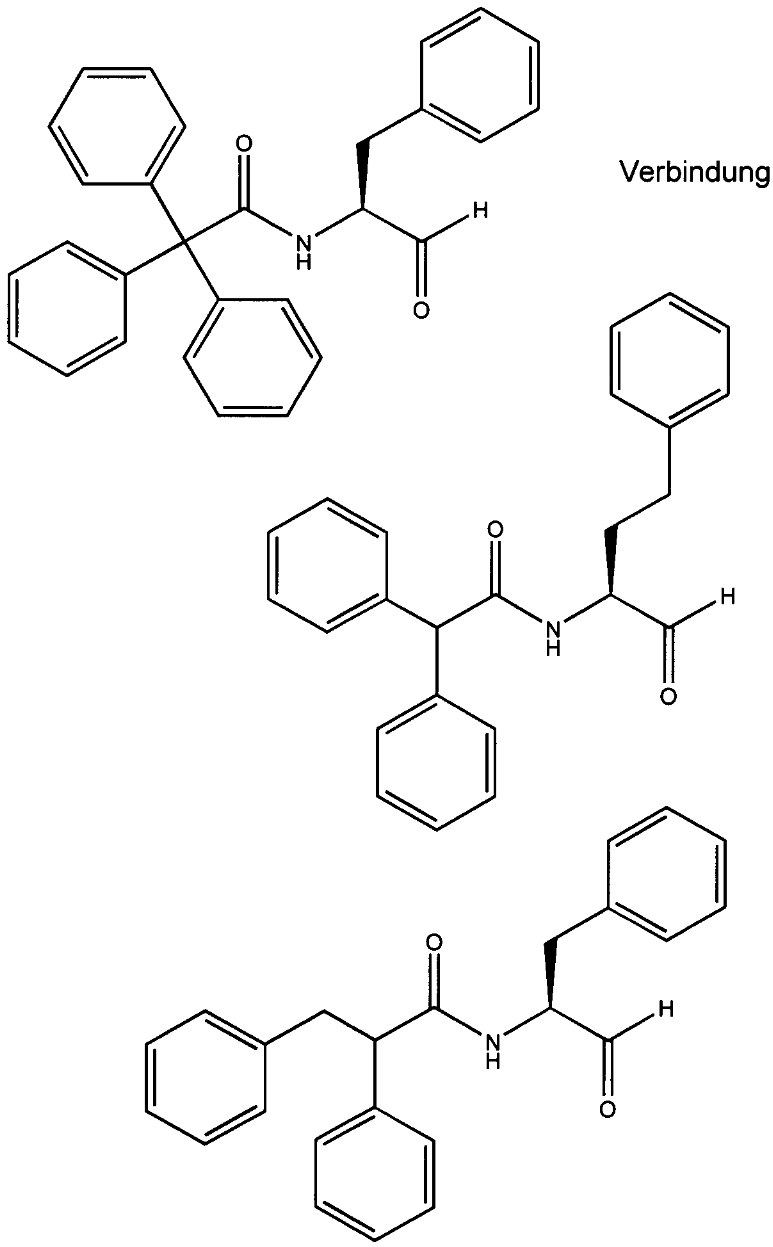

Verbindung 3

Verbindung 4

Verbindung 5

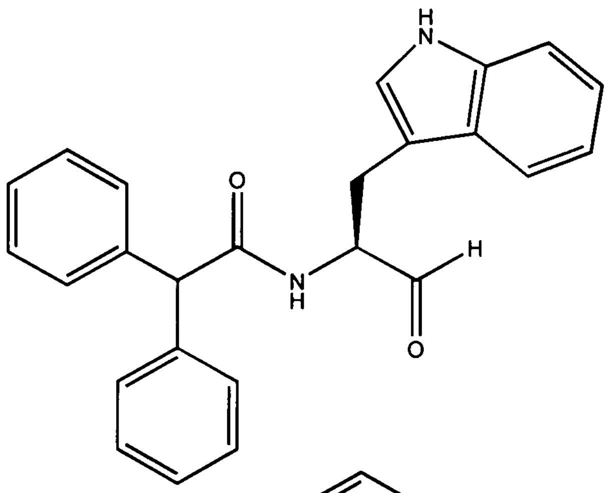

Verbindung 6

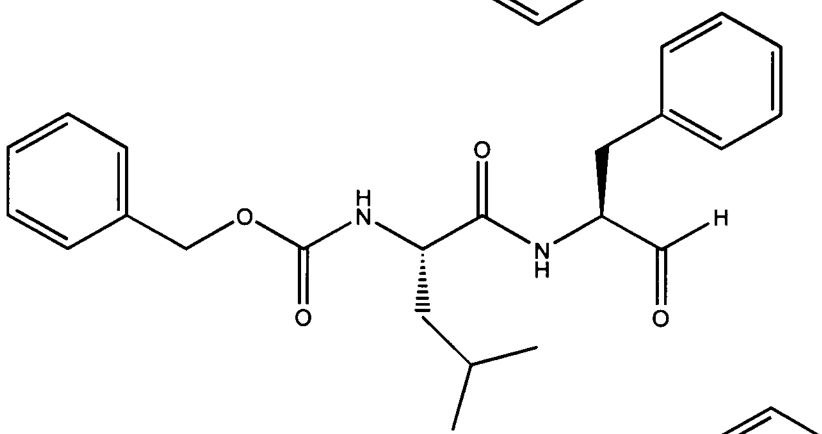

Verbindung 7

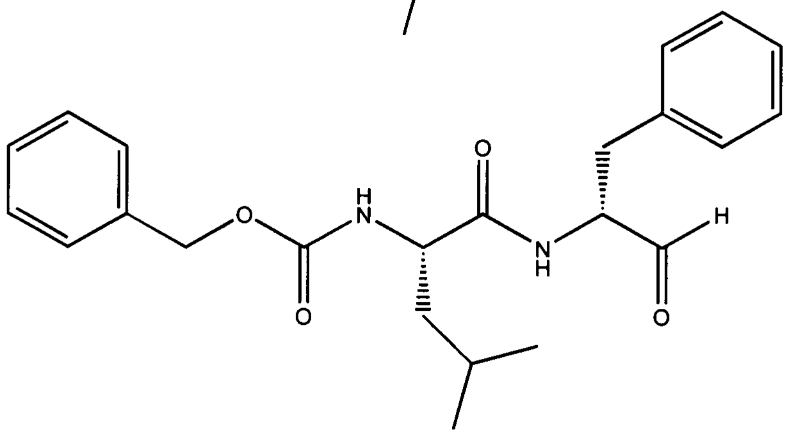

Verbindung 8

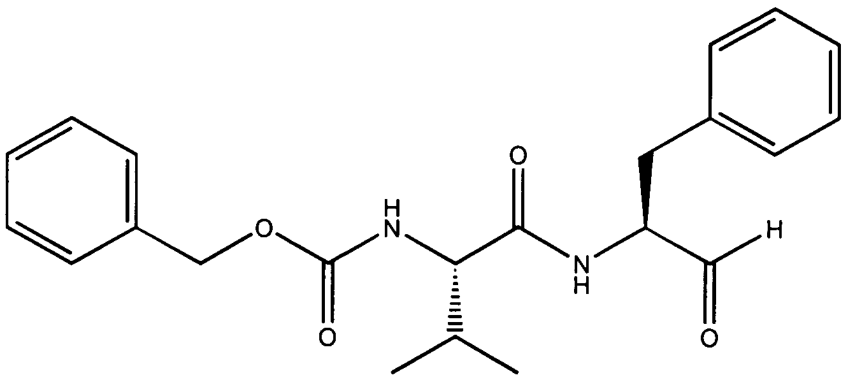

Verbindung 9

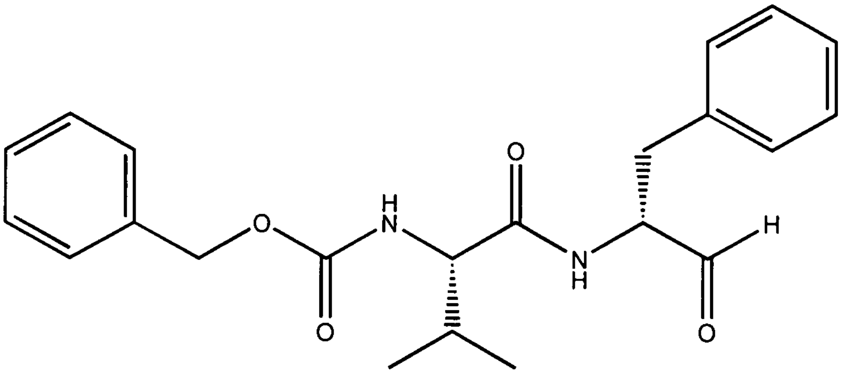

Verbindung 10

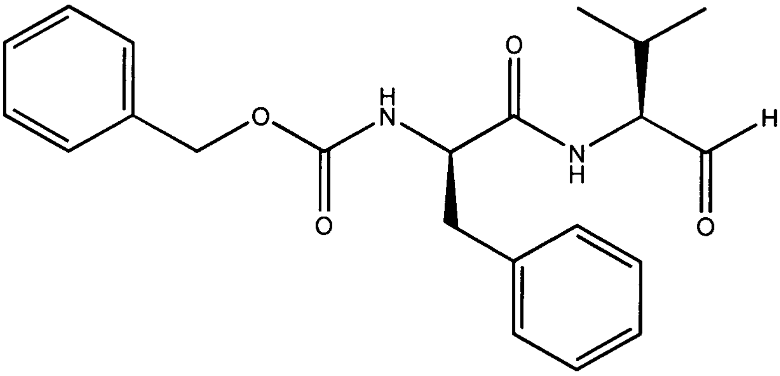

Verbindung 11

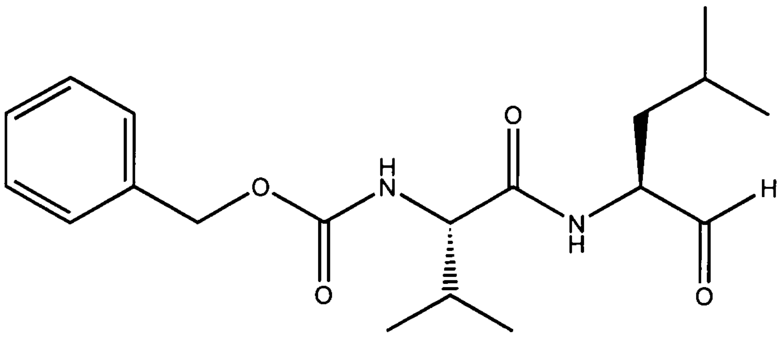

Verbindung 12

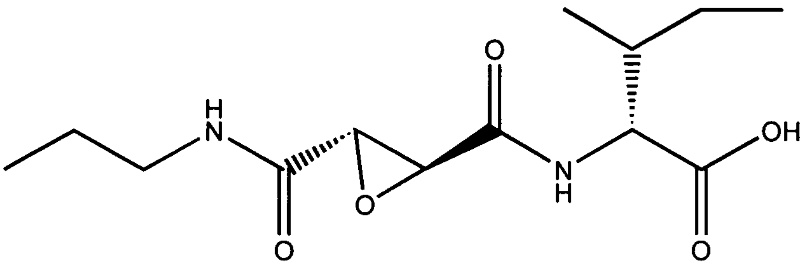

Verbindung 13

**7.** Verfahren zum Diagnostizieren eines Schmerzstatus außerhalb eines lebenden Körpers oder zur Unterstützung

der Wirksamkeit einer Schmerzbehandlung außerhalb eines lebenden Körpers, charakterisiert durch die Verwendung einer Polynucleotidsequenz, die Cathepsin Y kodiert, ausgewählt aus SEQ ID NO:1, 2 oder 4 oder Homologen, oder des entsprechenden Polypeptids, umfassend die Bestimmung der Wirksamkeit einer Verbindung oder eines Mittels durch Betrachten der Expressionsrate des Gens oder der Aktivität des Proteins.

**8.** Verfahren nach einem der Ansprüche 1 bis 4 und 7 oder Verwendung nach Anspruch 5 oder 6, wobei der Schmerz neuropathischer Schmerz ist.

**9.** Ein nichtmenschliches transgenes Tier, wobei das Gen, das Cathepsin Y entsprechend SEQ ID NO: 1, 2 oder 4 oder Homologe kodiert, in dem Tier manipuliert ist, was zu einem unterschiedlichen Expressiongrad oder einer unterschiedlichen Proteinaktivität im Vergleich zu dem Wildtyp führt.

## Revendications

**1.** Procédé pour le développement d'un médicament pour le traitement de la douleur, comportant l'utilisation d'une séquence de polynucléotides sélectionnée parmi SEQ ID N° : 1, 2 ou 4 encodant la cathepsine Y ou des homologues, ou la protéine/le polypeptide en accord, comportant la détermination de la quantité d'un composé ou agent dans un modèle pour la douleur, de sorte que l'efficacité du composé/de l'agent est déterminée en considérant le taux d'expression du gène, ou en considérant l'activité de la protéine.

**2.** Procédé selon la revendication 1, comprenant les étapes consistant à :

a) fournir une population de cellules de test comprenant des cellules capables d'exprimer les homologues de gène de cathepsine Y, ou des fragments de ceux-ci,
b) mettre en contact ladite population de cellules de test avec l'agent thérapeutique de test,
c) détecter l'expression du ou des gènes de cathepsine Y dans ladite population de cellules de test,
d) comparer l'expression du ou des gènes dans la population de cellules de test à l'expression du ou des gènes dans une population de cellules de référence dont le stade de maladie est connu ; et
e) identifier une différence des niveaux d'expression des séquences considérées, s'il y en a, dans la population de cellules de test et la population de cellules de référence, en identifiant ainsi un agent thérapeutique pour le traitement de la douleur.

**3.** Procédé selon la revendication 1 ou 2, comprenant les étapes consistant à

a) mettre en contact la protéine de cathepsine Y avec un composé/agent en présence d'un polypeptide qui est une cible de la cathepsine Y,
b) déterminer l'acide aminé amino-terminal du peptide ou la quantité d'acides aminés libres dans l'échantillon après incubation,
c) comparer l'acide aminé amino-terminal du peptide ou la quantité d'acides aminés libres avec le résultat d'un échantillon qui ne contient pas le composé/l'agent.

**4.** Procédé selon la revendication 2 ou 3, dans lequel l'expression génique est considérée ou déterminée par PCR d'ADNc, croisement d'un échantillon d'ADN, ou détection de la protéine en accord.

**5.** Utilisation d'un composé régulant l'activité de cathepsine Y encodée par SEQ ID N° : 1, 2 ou 4 pour la fabrication d'un médicament pour le traitement de la douleur, le composé ayant la formule générale

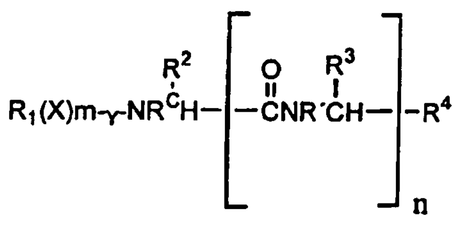

formule I

dans laquelle :

R est sélectionné dans le groupe comprenant un groupe hydrogène, alkyle ayant de 1 à 6 atomes de carbone, et dans laquelle R et $R^2$ sont reliés pour former une structure de noyau ayant de 4 à 10 atomes de carbone,
R' est sélectionné dans le groupe comprenant un groupe hydrogène, alkyle ayant de 1 à 6 atomes de carbone, et dans laquelle R' et $R^3$ sont reliés pour former une structure de noyau ayant de 4 à 6 atomes de carbone,
$R^1$ est sélectionné dans le groupe comprenant un groupe alkyle ayant de 1 à 4 atomes de carbone remplacés par 1 à 5 substituants sélectionnés dans le groupe comprenant (a) un groupe aryle ayant de 6 à 10 atomes de carbone, (b) un groupe aryle ayant de 6 à 10 atomes de carbone remplacés par 1 à 3 substituants sélectionnés dans le groupe comprenant un groupe alkyle ayant de 1 à 6 atomes de carbone, aryle ayant de 6 à 10 atomes de carbone, alcoxy ayant de 1 à 6 atomes de carbone, aryloxy ayant de 6 à 10 atomes de carbone, hydroxy, cyano, halo et amino, (c) cycloalkyle ayant de 3 à 8 atomes de carbone et (d) des hétérocycles ayant de 3 à 14 atomes de carbone, comportant de 1 à 3 hétéroatomes sélectionnés dans le groupe comprenant de l'azote, de l'oxygène et du souffre,
dans laquelle ledit groupe alkyle substitué est facultativement remplacé en outre par 1 à 2 groupes hydroxyle,
un groupe alcényle ayant de 2 à 4 atomes de carbone remplacés par 1 à 4 substituants sélectionnés dans le groupe comprenant (a) un groupe aryle ayant de 6 à 10 atomes de carbone, (b) un groupe aryle ayant de 6 à 10 atomes de carbone remplacés par 1 à 3 substituants sélectionnés dans le groupe comprenant un groupe alkyle ayant de 1 à 6 atomes de carbone, aryle ayant de 6 à 10 atomes de carbone, alcoxy ayant de 1 à 6 atomes de carbone, aryloxy ayant de 6 à 10 atomes de carbone, hydroxy, cyano, halo, amino, (c) cycloalkyle ayant de 3 à 8 atomes de carbone et (d) des hétérocycles ayant de 3 à 14 atomes de carbone, comportant de 1 à 3 hétéroatomes sélectionnés dans le groupe comprenant de l'azote, de l'oxygène et du souffre,
un groupe aryle ayant de 6 à 10 atomes de carbone,
un groupe aryle ayant de 6 à 10 atomes de carbone remplacés par 1 à 3 substituants sélectionnés dans le groupe comprenant un groupe alkyle ayant de 1 à 6 atomes de carbone, aryle ayant de 6 à 10 atomes de carbone, alcoxy ayant de 1 à 6 atomes de carbone, aryloxy ayant de 6 à 10 atomes de carbone, hydroxy, cyano, halo et amino,
un fluorényle,
des hétérocycles ayant de 3 à 14 atomes de carbone, comportant de 1 à 3 hétéroatomes sélectionnés dans le groupe comprenant de l'azote, de l'oxygène et du souffre ;
$R^2$ et $R^3$ sont indépendamment des chaînes latérales d'acides aminés D ou L d'au moins 2 atomes de carbone, pour autant que lesdites chaînes latérales d'acides aminés ne comprennent pas la chaîne latérale de proline ;
$R^4$ est sélectionné dans le groupe comprenant
$-C(O)CH=N=N$, $-CH_2OH$, $-C=NOH$, et $-C(O)R^5$
où $R^5$ est un groupe hydrogène, alkyle ayant de 1 à 6 atomes de carbone, halogénoalkyle ayant de 1 à 6 atomes de carbone et 1 à 2 groupes halo, alcoxy ayant de 1 à 6 atomes de carbone, $-NR^6R^7$, où $R^6$ et $R^7$ sont sélectionnés indépendamment dans le groupe comprenant un groupe hydrogène et alkyle ayant de 1 à 6 atomes de carbone, et aryle ayant de 6 à 10 atomes de carbone, et $-N(CH_3)OCH_3$ ;
X est sélectionné dans le groupe comprenant -O-, $-NR^9-$, et -S-, où $R^9$ est sélectionné dans le groupe comprenant un groupe hydrogène, alkyle ayant de 1 à 6 atomes de carbone et aryle ayant de 6 à 10 atomes de carbone ;
Y est sélectionné dans le groupe comprenant -C(O)- et -C(S)- ;
m est égal à zéro ou un ; et
n est égal à zéro, un ou deux,
ou des sels pharmaceutiquement acceptables de ceux-ci
pour autant que lorsque $R^1$ est 1-naphtyle, $R^2$ est $(CH(CH_3)_2$ (isomère L), $R^3$ est $-CH_2-\varnothing$ (isomère L), Y est -C(O)-, m est zéro et n est un, alors $R^4$ n'est pas $-N(CH_3)OCH_3$, avec la condition supplémentaire que lorsque R' est du diphénylméthyle, $R^2$ est du p-(benzyloxy)benzyle (isomère L), Y est -C(O)-, et m et n sont zéro, alors $R^4$ n'est pas $-N(CH_3)OCH_3$, et toujours avec la condition qui est que lorsque $R^1$ est du (1,2-diphényl)éthényle, Y est -C(O)-, $R^2$ est $-CH_2-\varnothing$ (isomère L), et m et n sont zéro, alors $R^4$ n'est pas $-N(CH_3)OCH_3$.

**6.** Utilisation selon la revendication 5,
dans laquelle le composé est sélectionné dans le groupe comprenant

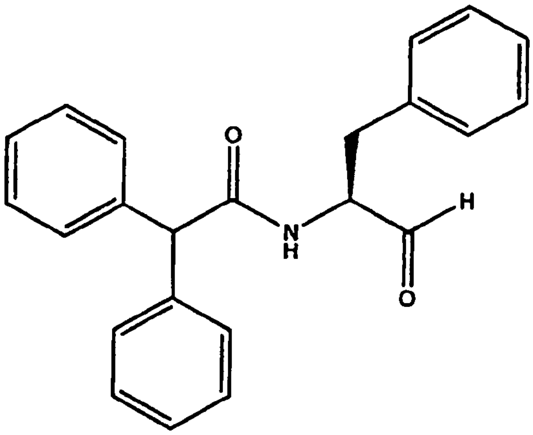

Composé 1

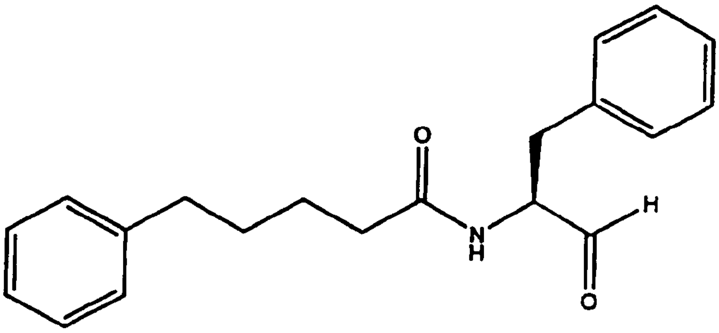

Composé 2

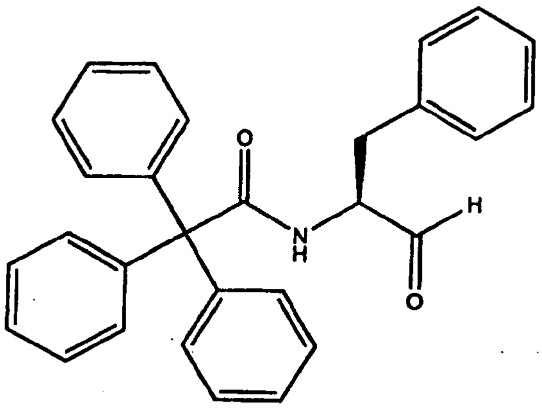

Composé 3

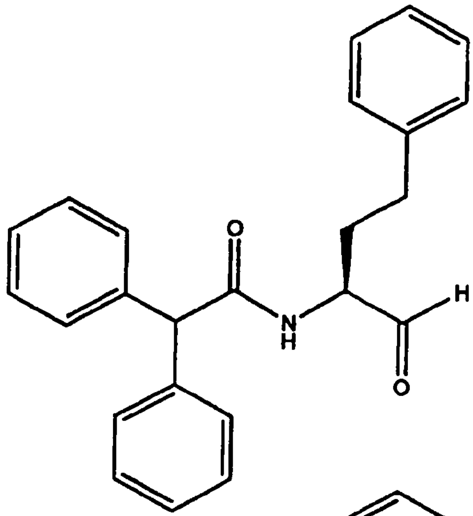

Composé 4

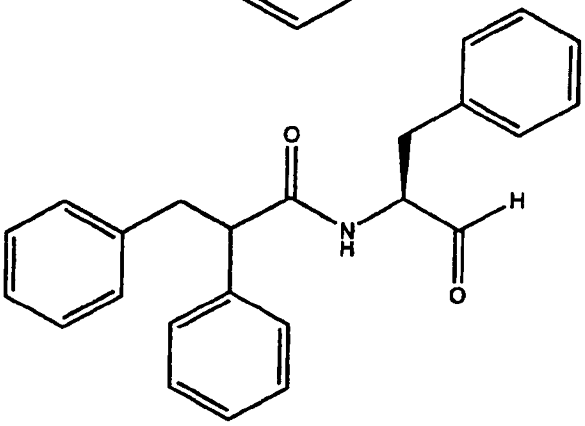

Composé 5

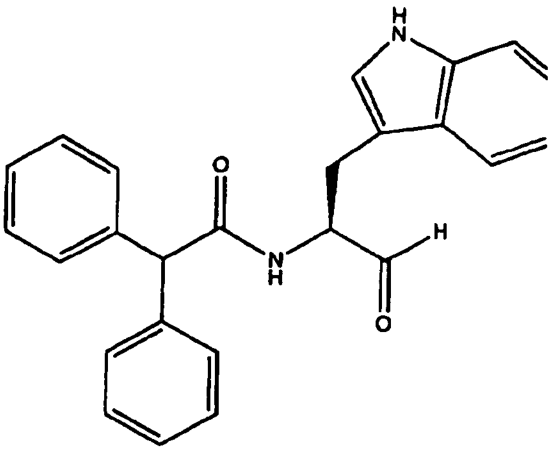

Composé 6

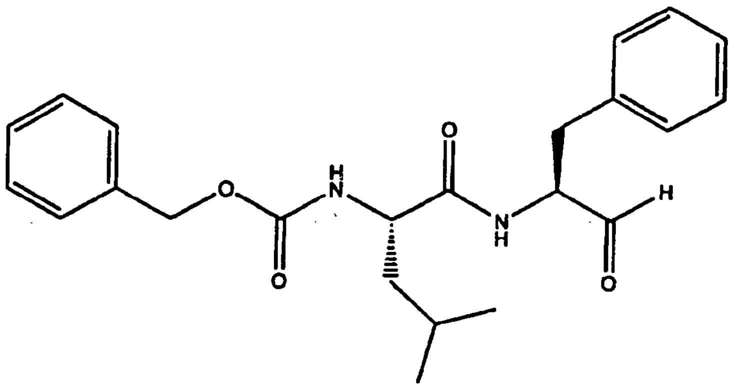

Composé 7

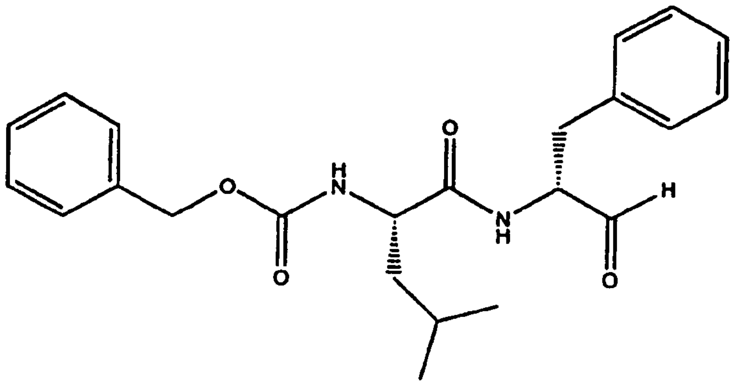

Composé 8

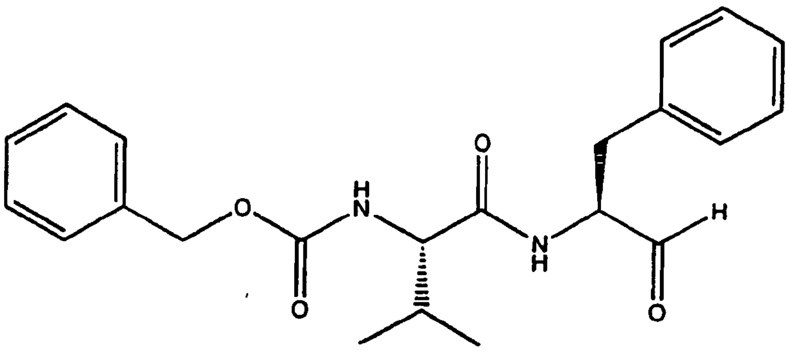

Composé 9

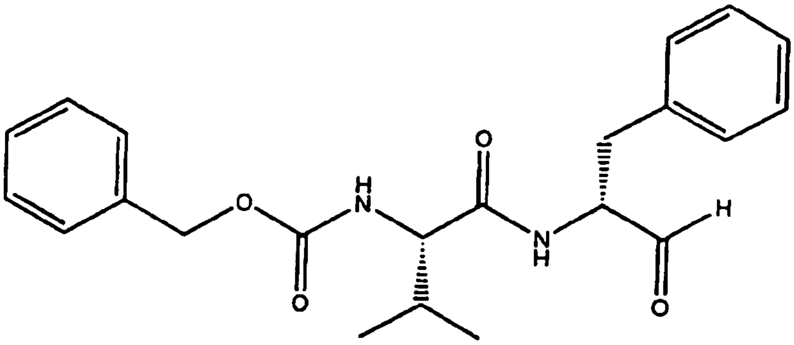

Composé 10

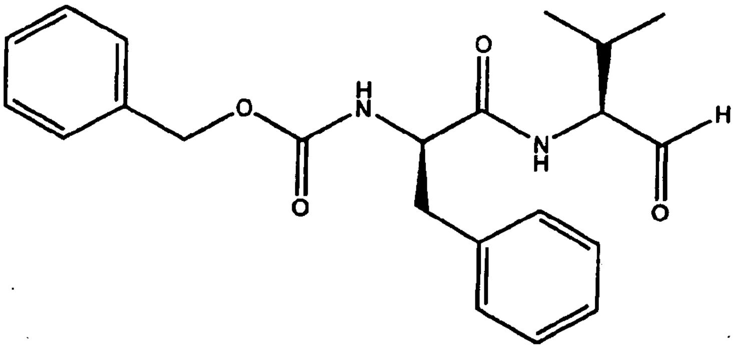

Composé 11

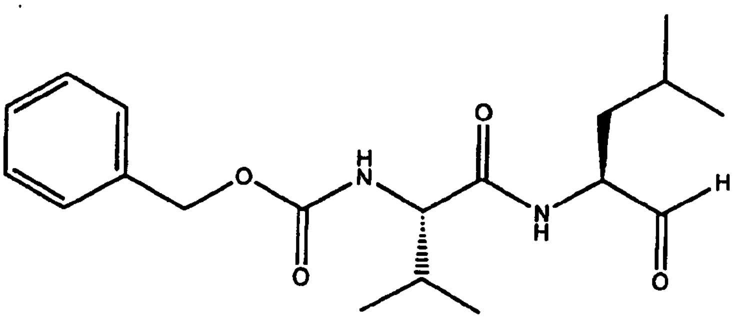

Composé 12

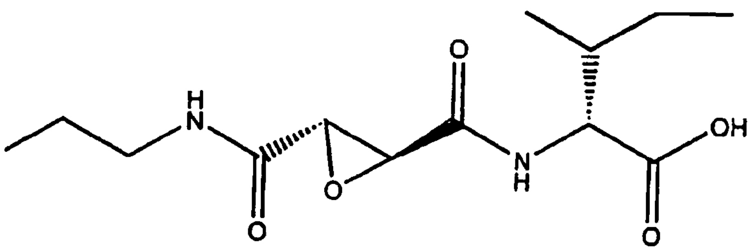

Composé 13

7. Procédé pour diagnostiquer un état de douleur à l'extérieur d'un corps vivant, ou pour évaluer l'efficacité d'un traitement contre la douleur à l'extérieur d'un corps vivant, **caractérisé par** l'utilisation d'une séquence de polynucléotides encodant la cathepsine Y sélectionnée parmi SEQ ID N° : 1, 2 ou 4 ou des homologues du polypeptide en accord, comportant la détermination de l'efficacité d'un composé ou agent en considérant le taux d'expression

du gène, ou en considérant l'activité de la protéine.

**8.** Procédé selon l'une quelconque des revendications 1 à 4 et 7, ou utilisation selon la revendication 5 ou 6, où la douleur est une douleur neuropathique.

**9.** Animal transgénique non humain, dans lequel le gène encodant la cathepsine Y selon SEQ ID N° : 1, 2 ou 4, ou des homologues, est manipulé dans l'animal, en ayant pour résultat un niveau d'expression différent ou une activité protéinique différente par comparaison au type d'animal.

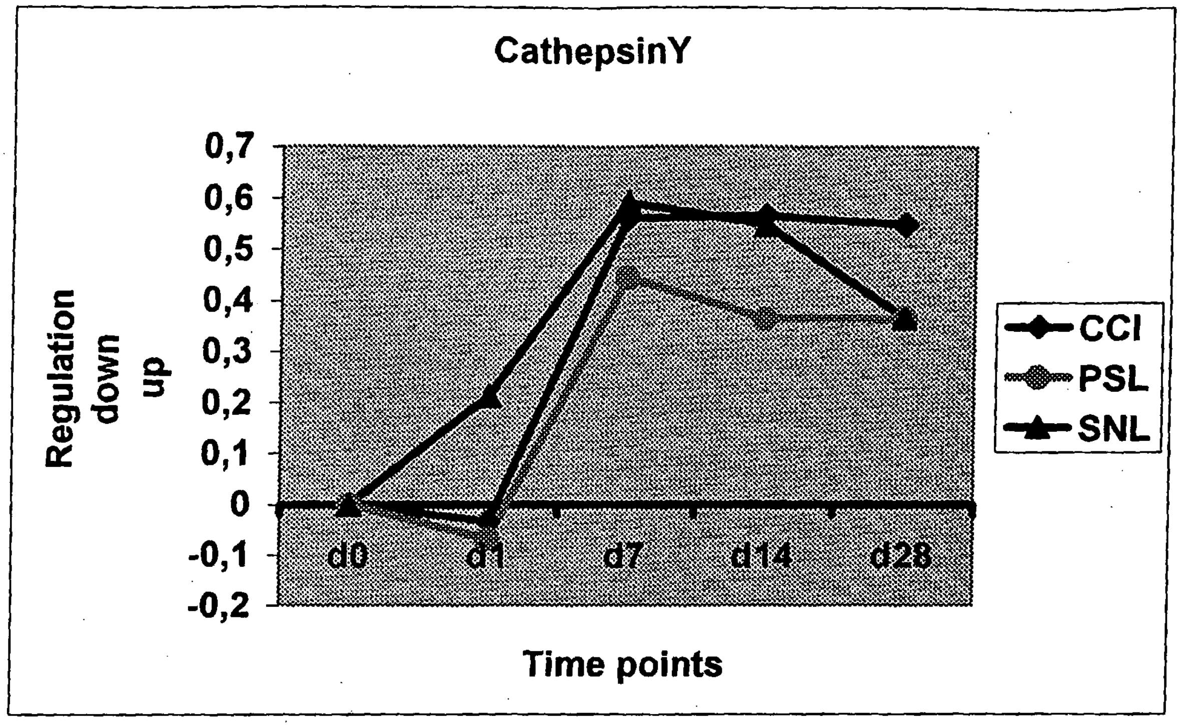
CathepsinY
0,7
0,6
0,5
0,4
0,3
0,2
0,1
0
-0,1
-0,2
Regulation
down
up
d0
d1
d7
d14
d28
Time points
CCI
PSL
SNL

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9931256 A
- WO 0123570 A
- JP 2000157263 B
- WO 9639194 A
- WO 9942610 A

### Non-patent literature cited in the description


- Principles of Neurology. McGraw-Hill, 1997, 133-134
- Principles of Neurology. McGraw-Hill, 1997, 140
- The Merck Manual. 1992, 1518-1522
- The Merck Manual. 1992, 1518
- **BENNETT ; XIE.** *Pain,* 1988, vol. 33, 87-107
- **SELTZER et al.** *Pain,* 1990, vol. 43, 205-218
- **KIM ; CHUNG.** *Pain,* 1992, vol. 50, 355-363
- **DELEO et al.** *Pain,* 1994, vol. 56, 9-16
- **NA et al.** *Neurosci. Lett.,* 1994, vol. 177, 50-52
- **KIM et al.** *Exp. Brain Res.,* 1997, vol. 113, 200-206
- **WALKER et al.** *Molecular Medicine Today,* 1999, vol. 5, 319-321
- **JAN DEUSSING et al.** *Biochimica et Biophysica Acta,* 2000, 93-106
- **PUNGERCAR, J. ; IVANOVSKI, G.** *Pfluegers Archiv European Journal of Physiology,* 2000, vol. 439 (3), R116-R118
- **NÄGLER, D. ; MÉNARD, R.** *FEBS Letters,* 1998, vol. 434, 135-139
- **THERRIEN, C. et al.** *Biochemistry,* 2001, vol. 40, 2702-2711
- **BENNETT.** *Pain,* 1988, vol. 33, 87-107
- **SELTZER ; DUBNER ; SHIR.** *Pain,* 1990, vol. 43, 205-218
- **ALTSCHUL et al.** *Nucleic Acids Res.,* 1997, vol. 25, 3389-3402
- **SIMONS et al.** *JACS,* 1976, vol. 98, 7098-7099